# EUROPEAN PATENT SPECIFICATION

(11) **EP 1 758 900 B1**
(45) Date of publication and mention of the grant of the patent: **03.10.2007**
(21) Application number: 05752619.6
(22) Date of filing: 07.06.2005
(51) Int. Cl.: C07D 471/04, A61K 31/435

(54) **SUBSTITUTED TRICYCLIC BENZIMIDAZOLES**
SUBSTITUIERTE TRICYCLISCHE BENZIMIDAZOLE
BENZIMIDAZOLES TRICYCLIQUES SUBSTITUES

(30) Priority: 09.06.2004 EP 04102628
(43) Date of publication of application: 07.03.2007
(73) Proprietor: Nycomed GmbH, 78467 Konstanz (DE)
(72) Inventor: BREHM, Christof, 68163 Mannheim (DE); ZIMMERMANN, Peter Jan, 71139 Ehningen (DE); BUHR, Wilm, 78465 Konstanz (DE); CHIESA, M. Vittoria, 78464 Konstanz (DE); PALMER, Andreas, 78224 Singen (DE); POSTIUS, Stefan, 78467 Konstanz (DE); KROMER, Wolfgang, 78464 Konstanz (DE); SIMON, Wolfgang-Alexander, 78464 Konstanz (DE)
(74) Representative: Wolf, Ulrich
(86) International application number: PCT/EP2005/052590
(87) International publication number: WO 2005/121139

(56) References cited:
- WO-A-03/014123
- WO-A-20/04087701
- US-B1- 6 384 048

## Description

### Technical field

The invention relates to novel compounds, which are used in the pharmaceutical industry as active compounds for the production of medicaments.

### Technical Background

In the international patent application WO 97/47603 (which corresponds to the US patent 6,465,505), benzimidazole derivatives having a very specific substitution pattern are disclosed, which are said to be suitable for inhibition of gastric acid secretion and thus can be used in the prevention and treatment of gastrointestinal inflammatory diseases.

In the European patent application EP 0266326 (which corresponds to US patent 5,106,862), benzimidazole derivatives having a very broad variety of substituents are disclosed, which are said to be active as antiulcer agents.

In the International patent applications WO 04/054984 and WO 04/087701 substituted benzimidazole derivatives are disclosed which compounds have gastric secretion inhibiting and excellent gastric and intestinal protective action properties.

### Summary of the invention

The invention relates to compounds of the formula 1 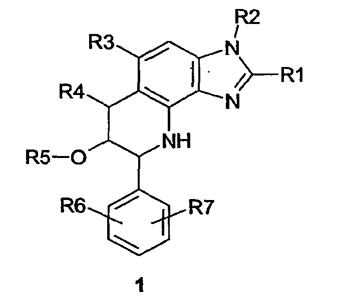 in which
- R1: is hydrogen, halogen, 1-4C-alkyl, 3-7C-cycloalkyl, 3-7C-cycloalkyl-1-4C-alkyl, 1-4C-alkoxy, 1-4C-alkoxy-1-4C-alkyl, 1-4C-alkoxycarbonyl, 2-4C-alkenyl, 2-4C-alkynyl, fluoro-1-4C-alkyl, fluoro-1-4C-alkoxy-1-4C-alkyl or hydroxy-1-4C-alkyl,
- R2: is hydrogen, 1-4C-alkyl, 3-7C-cycloalkyl, 3-7C-cycloalkyl-1-4C-alkyl, 1-4C-alkoxy-1-4C-alkyl, 2-4C-alkenyl, 2-4C-alkynyl, fluoro-1-4C-alkyl or hydroxy-1-4C-alkyl,
- R3: is halogen, fluoro-1-4C-alkyl, carboxyl, -CO-1-4C-alkoxy, hydroxy-1-4C-alkyl, 1-4C-alkoxy-1-4C-alkyl, 1-4C-alkoxy-1-4C-alkoxy-1-4C-alkyl, fluoro-1-4C-alkoxy-1-4C-alkyl, cyano, the group -CO-NR31 R32, the group SO₂-NR31 R32 or the group Het,
where
R31 is hydrogen, hydroxyl, 1-7C-alkyl, hydroxy-1-4C-alkyl, 1-4C-alkoxy-1-4C-alkyl, 3-7C-cycloalkyl, or amino and
R32 is hydrogen, 1-7C-alkyl, hydroxy-1-4C-alkyl or 1-4C-alkoxy-1-4C-alkyl, or where
R31 and R32 together, including the nitrogen atom to which both are bonded, are a pyrrolidino, piperidino, piperazino, N-1-4.Galkylpiperazino, morpholino, aziridino or azetidino group and Het is a heterocyclic residue, substituted by R33, R34 and R35, selected from the group consisting of oxadiazol, dihydrooxazol, dihydroimidazol, oxazol, imidazol, isoxazol, dihydroisoxazol, pyrazol and tetrazol,
where
R33 is hydrogen, 1-4C-alkyl, hydroxy-1-4C-alkyl, 1-4C-alkoxy, 2-4C-alkenyloxy, 1-4C-alkylcarbonyl, carboxy, 1-4C-alkoxycarbonyl, carboxy-1-4C-alkyl, 1-4C-alkoxycarbonyl-1-4C-alkyl, halogen, hydroxy, aryl, aryl-1-4C-alkyl, aryl-oxy, aryl-1-4C-alkoxy, trifluoromethyl, nitro, amino, mono- or di-1-4C-alkylamino, 1-4C-alkylcarbonylamino, 1-4C-alkoxycarbonylamino, 1-4C-alkoxy-1-4C-alkoxycarbonylamino or sulfonyl,
R34 is hydrogen, 1-4C-alkyl, 1-4C-alkoxy, 1-4C-alkoxycarbonyl, halogen, trifluoromethyl or hydroxy,
R35 is hydrogen, 1-4C-alkyl, 1-4C-alkoxy, 1-4C-alkoxycarbonyl, halogen, trifluoromethyl or hydroxy,
- R4: is hydrogen, halogen, 1-4C-alkyl, 3-7C-cycloalkyl, 3-7C-cycloalkyl-1-4C-alkyl. 1-4C-alkoxy-1-4C-alkyl, fluoro-1-4C-alkyl, fluoro-1-4C-alkoxy-1-4C-alkyl, hydroxy-1-4C-alkyl, hydroxy, 1-4C-alkoxy, 3-7C-cycloalkoxy, 3-7C-cycloalkyl-1-4C-alkoxy, 1-4C-alkoxy-1-4C-atkoxy, fluoro-1-4C-alkoxy, fluoro-1-4C-alkoxy-1-4C-alkoxy, hydroxy-1-4C-alkoxy, 2-4C-alkenyloxy or 2-4C-alkynyloxy
- R5: is hydrogen, 1-4C-alkyl, 3-7C-cycloalkyl, 3-7C-cycloalkyl-1-4C-alkyl, 1-4C-alkoxy-1-4C-alkyl, fluoro-1-4C-alkyl, fluoro-1-4C-alkoxy- 1-4C-alkyl or hydroxy-1-4C-alkyl,
- R6: is hydrogen, halogen, 1-4C-alkyl or fluoro-1-4C-alkyl,
- R7: is hydrogen, halogen, 1-4C-alkyl or fluoro-1-4C-alkyl,
and wherein
- aryl: is phenyl or substituted phenyl with one, two or three identical or different substituents from the group of 1-4C-alkyl, 1-4C-alkoxy, carboxy, 1-4C-alkoxycarbonyl, halogen, trifluoromethyl, nitro, trifluoromethoxy, hydroxy and cyano,
and the salts of these compounds.

Halogen within the meaning of the invention is bromo, chloro and fluoro.

1-4C-Alkyl represents a straight-chain or branched alkyl group having 1 to 4 carbon atoms. Examples which may be mentioned are the butyl, isobutyl, sec-butyl, tert-butyl, propyl, isopropyl, ethyl and the methyl group.

3-7C-Cycloalkyl represents cyclopropyl, cyclobutyl, cyclopentyl, cyclohexyl and cycloheptyl, of which cyclopropyl, cyclobutyl and cyclopentyl are preferred.

3-7C-Cycloalkyl-1-4C-alkyl represents one of the aforementioned 1-4C-alkyl groups, which is substituted by one of the aforementioned 3-7C-cycloalkyl groups. Examples which may be mentioned are the cyclopropylmethyl, the cyclohexylmethyl and the cyclohexylethyl group.

1-4C-Alkoxy represents a group, which in addition to the oxygen atom contains one of the aforementioned
1-4C-alkyl groups. Examples which may be mentioned are the butoxy, isobutoxy, sec-butoxy, tert-butoxy, propoxy, isopropoxy and preferably the ethoxy and methoxy group.

1-4C-Alkoxy-1-4C-alkyl represents one of the aforementioned 1-4C-alkyl groups, which are substituted by one of the aforementioned 1-4C-alkoxy groups. Examples which may be mentioned are the methoxymethyl, the methoxyethyl group and the butoxyethyl group.

1-4C-Alkoxycarbonyl (-CO-1-4C-alkoxy) represents a carbonyl group, to which one of the aforementioned 1-4C-alkoxy groups is bonded. Examples which may be mentioned are the methoxycarbonyl (CH₃O-C(O)-) and the ethoxycarbonyl group (CH₃CH₂O-C(O)-).

2-4C-Alkenyl represents a straight-chain or branched alkenyl group having 2 to 4 carbon atoms. Examples which may be mentioned are the 2-butenyl, 3-butenyl, 1-propenyl and the 2-propenyl group (allyl group).

2-4C-Alkynyl represents a straight-chain or branched alkynyl group having 2 to 4 carbon atoms. Examples which may be mentioned are the 2-butynyl, 3-butynyl, and preferably the 2-propynyl, group (propargyl group).

Fluoro-1-4C-alkyl represents one of the aforementioned 1-4C-alkyl groups, which is substituted by one or more fluorine atoms. Examples which may be mentioned are the trifluoromethyl or the difluoromethyl group.

Fluoro-1-4C-alkoxy represents a group, which in addition to the oxygen atom contains one of the aforementioned fluoro-1-4C-alkyl groups. Examples which may be mentioned are the 1,1,1,3,3,3-hexafluoro-2-propoxy, the 2-trifluoromethyl-2-propoxy, the 1,1,1-trifluoro-2-propoxy, the perfluoro-tert-butoxy, the 2,2,3,3,4,4,4-heptafluoro-1-butoxy, the 4,4,4-trifluoro-1-butoxy, the 2,2,3,3,3-pentafluoropropoxy, the perfluoroethoxy, the 1,2,2-trifluoroethoxy, in particular the 1,1,2,24etrafluoroethoxy, the 2,2,2-trifluoroethoxy, the trifluoromethoxy and preferably the difluoromethoxy, the 2,2,2-trifluoroethoxy, the 2,2-difluoroethoxy and the 2-fluoroethoxy group.

Fluoro-1-4C-alkoxy-1-4C-alkyl represents one of the aforementioned 1-4C-alkyl groups, which is substituted by a fluoro-1-4C-alkoxy group. Examples of fluoro-1-4C-alkoxy-1-4C-alkyl groups are the 1,1,2,2-tetrafluoroethoxymethyl, the 2,2,2-trifluoroethoxymethyl, the trifluoromethoxyethyl and the difluoromethoxyethyl group.

Hydroxy-1-4C-alkyl represents one of the aforementioned 1-4C-alkyl groups, which is substituted by a hydroxy group. Examples which may be mentioned are the hydroxymethyl, the 2-hydroxyethyl and the 3-hydroxypropyl group.

3-7C-Cycloalkoxy represents a group, which in addition to the oxygen atom contains one of the aforementioned 3-7C-cycloalkyl groups. Examples which may be mentioned are the cyclopropyloxy, cyclobutyloxy, cyclopentyloxy, cyclohexyloxy and cycloheptyloxy, of which cyclopropyloxy, cydobutyloxy and cyclopentyloxy are preferred.

1-4C-Alkoxy-1-4Galkoxy represents one of the aforementioned 1-4C-alkoxy groups, which is substituted by one of the aforementioned 1-4C-alkoxy groups. Examples which may be mentioned are the 2-methoxyethoxy, the 2-ethoxyethoxy group and the 2-butoxyethoxy group.

1-4C-Alkoxy-1-4C-alkoxy-1-4C-alkyl represents one of the aforementioned 1-4C-alkoxy-1-4C-alkyl groups, which is substituted by one of the aforementioned 1-4C-alkoxy groups. An example which may be mentioned is the group 2-(methoxy)ethoxymethyl (CH₃-O-CH₂-CH₂-O-CH₂-).

1-7C-Alkyl represents straight-chain or branched alkyl groups having 1 to 7 carbon atoms. Examples which may be mentioned are the heptyl, isoheptyl (5-methylhexyl), hexyl, isohexyl (4-methylpentyl), neohexyl (3,3-dimethylbutyl), pentyl, isopentyl (3-methylbutyl), neopentyl (2,2-dimethylpropyl), butyl, isobutyl, sec-butyl, tert-butyl, propyl, isopropyl, ethyl and the methyl group.

2-4C-Alkenyloxy represents groups, which in addition to the oxygen atom contain one of the abovementioned 2-4C-alkenyl groups. Examples, which may be mentioned, are the 2-butenyloxy, 3-butenyloxy, 1-propenyloxy and the 2-propenyloxy group (allyloxy group).

1-4C-Alkylcarbonyl represents a group, which in addition to the carbonyl group contains one of the aforementioned 1-4C-alkyl groups. An example which may be mentioned is the acetyl group.

Carboxy-1-4C-alkyl represents 1-4C-alkyl groups which are substituted by a carboxyl group. Examples, which may be mentioned, are the carboxymethyl and the 2-carboxyethyl group.

1-4C-Alkoxycarbonyl-1-4C-alkyl represents 1-4C-alkyl groups, which are substituted by one of the abovementioned 1-4C-alkoxycarbonyl groups. Examples, which may be mentioned, are the Methoxycarbonylmethyl and the ethoxycarbonylmethyl group.

Aryl-1-4C-alkyl denotes an aryl-substituted 1-4C-alkyl radical. An example which may be mentioned is the benzyl radical.

Aryl-1-4C-alkoxy denotes an aryl-substituted 1-4C-alkoxy radical. An example which may be mentioned is the benzyloxy radical.

Mono- or di-1-4C-alkylamino represents an amino group, which is substituted by one or by two - identical or different - groups from the aforementioned 1-4C-alkyl groups. Examples which may be mentioned are the dimethylamino, the diethylamino and the diisopropylamino group.

1-4C-Alkylcarbonylamino represents an amino group to which a 1-4C-alkylcarbonyl group is bonded. Examples which may be mentioned are the propionylamino (C₃H₇C(O)NH-) and the acetylamino group (acetamido group) (CH₃C(O)NH-) .

1-4C-Alkoxycarbonylamino represents an amino group, which is substituted by one of the aforementioned 1-4C-alkoxycarbonyl groups. Examples, which may be mentioned, are the ethoxycarbonylamino and the methoxycarbonylamino group.

1-4G-Alkoxy-1-4C-alkoxycarbonyl represents a carbonyl group, to which one of the aforementioned 1-4C-alkoxy-1-4C-alkoxy groups is bonded. Examples which may be mentioned are the 2-(methoxy)-ethoxycarbonyl (CH₃-O-CH₂CH₂-O-CO-) and the 2-(ethoxy)ethoxycarbonyl group (CH₃CH₂-O-CH₂CH₂-O-CO-).

1-4C-Alkoxy-1-4C-alkoxycarbonylamino represents an amino group, which is substituted by one of the aforementioned 1-4C-alkoxy-1-4C-alkoxycarbonyl groups. Examples which may be mentioned are the 2-(methoxy)ethoxycarbonylamino and the 2-(ethoxy)ethoxycarbonylamino group.

3-7C-Cycloalkyl-1-4C-alkoxy represents one of the aforementioned 1-4C-alkoxy groups, which is substituted by one of the aforementioned 3-7C-cycloalkyl groups. Examples which may be mentioned are the cyclopropylmethoxy, the cyclohexylmethoxy and the 2-cyclohexylethoxy group.

Fluoro-1-4C-alkoxy-1-4C-alkoxy represents one of the aforementioned 1-4C-alkoxy groups, which is substituted by a fluoro-1-4C-alkoxy group. Examples of fluoro-1-4C-alkoxy-1-4C-alkoxy groups are the 1,1,2,2-tetrafluoroethoxy-2-ethoxy, the 2,2,2-trifluoroethoxy-2-ethoxy, the trifluoromethoxy-2-ethoxy and the difluoromethoxy-2-ethoxy group.

Hydroxy-1-4C-alkoxy represents one of the aforementioned 1-4C-alkoxy groups, which is substituted by a hydroxy group. Examples which may be mentioned are the 2-hydroxyethoxy and the 3-hydroxypropoxy group.

2-4C-Alkynyloxy represents groups, which in addition to the oxygen atom contain one of the abovementioned 2-4C-alkynyl groups. Examples, which may be mentioned, are the 3-butynyloxy, 2-butynyloxy or the 2-propynyloxy group.

Possible salts of compounds of the formula 1 - depending on substitution - are especially all acid addition salts. Particular mention may be made of the pharmacologically tolerable salts of the inorganic and organic acids customarily used in pharmacy. Those suitable are water-soluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)benzoic acid, butyric acid, sulfosalicylic acid, maleic acid, lauric acid, malic acid, fumaric acid, succinic acid, oxalic acid, tartaric acid, embonic acid, stearic acid, toluenesulfonic acid, methanesulfonic acid or 3-hydroxy-2-naphthoic acid, where the acids are used in salt preparation - depending on whether a mono- or polybasic acid is concerned and on which salt is desired - in an equimolar quantitative ratio or one differing therefrom.

Pharmacologically intolerable salts, which can initially be obtained, for example, as process products in the production of the compounds according to the invention on the industrial scale, are converted into the pharmacologically tolerable salts by processes known to the person skilled in the art.

It is known to the person skilled in the art that the compounds according to invention and their salts, if, for example, they are isolated in crystalline form, can contain various amounts of solvents. The invention therefore also comprises all solvates and in particular all hydrates of the compounds of the formula 1, and also all solvates and in particular all hydrates of the salts of the compounds of the formula 1.

The compounds of the formula 1 have at least three centers of chirality in the skeleton. The invention thus provides all feasible stereoisomers in any mixing ratio, including the pure stereoisomers, which are a preferred subject matter of the invention.

One special aspect of the invention (aspect a) relates to compounds of the formula 1 wherein
- R1: is 1-4C-alkyl
and where R2, R3, R4, R5, R6 and R7 have the meanings as indicated in the outset.

Another special aspect of the invention (aspect b) relates to compounds of the formula 1 wherein
- R2: is 1-4C-alkyl,
and where R1, R3, R4, R5, R6 and R7 have the meanings as indicated in the outset.

Another special aspect of the invention (aspect c) relates to compounds of the formula 1 wherein
- R3: is the group -CO-NR31R32,
where
R31 is hydrogen, hydroxyl, 1-7C-alkyl, hydroxy-1-4C-alkyl, 1-4C-alkoxy-1-4C-alkyl or 3-7C-cycloalkyl and
R32 is hydrogen, 1-7C-alkyl, hydroxy-1-4C-alkyl or 1-4C-alkoxy-1-4C-alkyl,
or where
R31 and R32 together, including the nitrogen atom to which both are bonded, are a pyrrolidino, piperidino, piperazino, N-1-4C-alkylpiperazino, morpholino, aziridino or azetidino group,
and where R1, R2, R4, R5, R6 and R7 have the meanings as indicated in the outset.

Compounds which are to be mentioned are those of the formula 1 where
- R1: is hydrogen, 1-4C-alkyl or 3-7C-cycloalkyl,
- R2: is hydrogen or 1-4C-alkyl,
- R3: is halogen, Ouoro-1-4C-alkyl, carboxyl, -CO-1 -4C-alkoxy, hydroxy-1-4C-alkyl, 1-4C-alkoxy-1-4C-alkyl, 1-4C-alkoxy-1-4C-alkoxy-1-4C-alkyl, fluoro-1-4C-alkoxy-1-4C-alkyl or the group -CO-NR31 R32,
where
R31 is hydrogen, hydroxyl, 1-7C-alkyl, hydroxy-1-4C-alkyl, 1-4C-alkoxy-1-4C-alkyl or 3-7C-cycloalkyl and
R32 is hydrogen, 1-7C-alkyl, hydroxy-1-4C-alkyl or 1-4C-alkoxy-1-4C-alkyl,
or where
R31 and R32 together, including the nitrogen atom to which both are bonded, are a pyrrolidino, piperidino, piperazino, N-1-4C-alkylpiperazino, morpholino, aziridino or azetidino group,
- R4: is hydrogen, halogen, hydroxyl, 1-4C-alkyl, 3-5C-cycloalkyl, 1-4C-alkoxy, 3-5C-cycloalkoxy, 1-4C-alkoxy-1-4C-alkoxy, fluoro-1-4C-alkoxy, fluoro-1-4C-alkoxy-1-4C-alkoxy, hydroxy-1-4C-alkoxy, 2-4C-alkenyloxy or 2-4C-alkynyloxy, and
- R5: is hydrogen, 1-4C-alkyl or hydroxy-1-4C-alkyl,
- R6: is hydrogen, fluoro, 1-4C-alkyl or fluoro-1-4C-alkyl,
- R7: is hydrogen, fluoro, 1-4C-alkyl or fluoro-1-4C-alkyl,
and the salts of these compounds.

Compounds which are to be particularly mentioned are those of the formula 1 where
- R1: is hydrogen or 1-4C-alkyl,
- R2: is 1-4C-alkyl,
- R3: is carboxyl, -CO-1-4C-alkoxy, hydroxy-1-4C-alkyl, 1-4C-alkoxy-1-4C-alkyl or the group -CO-NR31R32,
where
R31 is hydrogen, 1-7C-alkyl, hydroxy-1-4C-alkyl, or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1 -4C-alkoxy, 2-4C-alkenyloxy or 2-4C-alkynyloxy
- R5: is hydrogen,
- R6: is hydrogen,
- R7: is hydrogen
and the salts of these compounds.

Compounds which are also to be particularly mentioned are those of the formula 1 where
- R1: is hydrogen or 1-4C-alkyl,
- R2: is 1-4C-alkyl,
- R3: is carboxyl, -CO-1-4C-alkoxy, hydroxy-14C-alkyl, 1-4C-alkoxy-1-4C-alkyl or the group -CO-NR31R32,
where
R31 is hydrogen, 1-7C-alkyl, hydroxy-1-4C-alkyl or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4Galkoxy, 2-4C-alkenyloxy or 2-4C-alkynyloxy
- R5: is hydrogen or 1-4C-alkyl,
- R6: is hydrogen,
- R7: is hydrogen
and the salts of these compounds.

Compounds which are to be emphasized are those of the formula 1,
in which
- R1: is hydrogen or 1-4C-alkyl,
- R2: is 1-4C-alkyl,
- R3: is carboxyl, -CO-1-4C-alkoxy or the group -CO-NR31 R32,
where
R31 is hydrogen, 1-4C-alkyl, hydroxy-1-4C-alkyl or 1-4C-alkoxy-1-4C-alkyl and
R32 is hydrogen or 1-4C-alkyl,
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy
- R5: is hydrogen,
- R6: is hydrogen,
- R7: is hydrogen
and the salts of these compounds.

Compounds which are also to be emphasized are those of the formula 1,
in which
- R1: is hydrogen or 1-4C-alkyl,
- R2: is 1-4C-alkyl,
- R3: is carboxyl, -CO-1-4C-alkoxy or the group -CO-NR31R32,
where
R31 is hydrogen, 1-4C-alkyl, hydroxy-1-4C-alkyl or 1-4C-alkoxy-1-4C-alkyl and
R32 is hydrogen or 1-4C-alkyl,
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy
- R5: is hydrogen or 1-4C-alkyl,
- R6: is hydrogen,
- R7: is hydrogen
and the salts of these compounds.

Compounds which are further to be emphasized are those of the formula 1,
in which
- R1: is hydrogen or 1-4C-alkyl,
- R2: is 1-4C-alkyl,
- R3: is -CO-1-4C-alkoxy or the group -CO-NR31 R32,
where
R31 is hydrogen or 1-4C-alkyl,
R32 is hydrogen or 1-4C-alkyl,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy
- R5: is hydrogen,
- R6: is hydrogen,
- R7: is hydrogen
and the salts of these compounds.

Compounds which are also further to be emphasized are those of the formula 1,
in which
- R1: is hydrogen or 1-4C-alkyl,
- R2: is 1-4C-alkyl,
- R3: is -CO-1-4C-alkoxy or the group -CO-NR31 R32,
where
R31 is hydrogen or 1-4C-alkyl,
R32 is hydrogen or 1-4C-alkyl,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy
- R5: is hydrogen or 1-4C-alkyl,
- R6: is hydrogen,
- R7: is hydrogen
and the salts of these compounds.

Compounds which are to be particularly emphasized are those of the formula 1,
in which
- R1: is 1-4C-alkyl,
- R2: is 1-4C-alkyl,
- R3: is -CO-NR31 R32,
where
R31 is hydrogen or 1-4C-alkyl
R32 is hydrogen or 1-4C-alkyl,
- R4: is hydrogen, hydroxyl, 1-4C-alkoxy, fluoro-1-4C-alkoxy, 1-4C-alkoxy-1-4C-alkoxy or 2-4C-alkynyloxy,
- R5: is hydrogen,
- R6: is hydrogen,
- R7: is hydrogen
and the salts of these compounds.

Compounds which are also to be particularly emphasized are those of the formula 1,
in which
- R1: is 1-4C-alkyl,
- R2: is 1-4C-alkyl,
- R3: is -CO-NR31 R32,
where
R31 is hydrogen or 1-4C-alkyl
R32 is hydrogen or 1-4C-alkyl,
- R4: is hydrogen, hydroxyl, 1-4C-alkoxy, fluoro-1-4C-alkoxy, 1-4C-alkoxy-1-4C-alkoxy or 2-4C-alkynyloxy,
- R5: is hydrogen or 1-4C-alkyl,
- R6: is hydrogen,

- R7: is hydrogen
and the salts of these compounds.

One embodiment (embodiment a) of the invention to be emphasized are compounds of the formula 1a 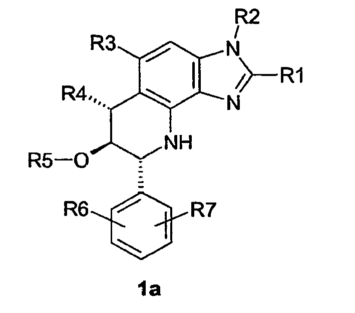 in which
- R1: is hydrogen, halogen, 1-4C-alkyl, 3-7C-cycloalkyl, 3-7C-cycloalkyl-1-4C-alkyl, 1-4C-alkoxy, 1-4C-alkoxy-1-4C-alkyl, 1-4C-alkoxycarbonyl, 2-4C-alkenyl, 2-4C-alkynyl, fluoro-1-4C-alkyl, fluoro-1-4C-alkoxy-1-4C-alkyl or hydroxy-1-4C-alkyl,
- R2: is hydrogen, 1-4C-alkyl, 3-7C-cycloalkyl, 3-7C-cycloalkyl-1-4C-alkyl, 1-4C-alkoxy- 1-4C-alkyl, 2-4C-alkenyl, 2-4C-alkynyl, fluoro-1-4C-alkyl or hydroxy-1-4C-alkyl,
- R3: is halogen, fluoro-1-4C-alkyl, carboxyl, -CO-1-4C-alkoxy, hydroxy-1-4C-alkyl, 1-4C-alkoxy-1-4C-alkyl, 1-4C-alkoxy-1-4C-alkoxy-1-4C-alkyl, fluoro-1-4Galkoxy-1-4Galkyl, cyano, the group -CO-NR31 R32, the group SO₂-NR31R32 or the group Het,
where
R31 is hydrogen, hydroxyl, 1-7C-alkyl, hydroxy-1-4C-alkyl, 1-4C-alkoxy-1-4C-alkyl 3-7C-cycloalkyl or amino and
R32 is hydrogen, 1-7C-alkyl, hydroxy-1-4C-alkyl or 1-4C-alkoxy-1-4C-alkyl,
or where
R31 and R32 together, including the nitrogen atom to which both are bonded, are a pyrrolidino, piperidino, piperazino, N-1-4C-alkylpiperazino, morpholino, aziridino or azetidino group and Het is a heterocyclic residue, substituted by R33, R34 and R35, selected from the group consisting of oxadiazol, dihydrooxazol, dihydroimidazol, oxazol, imidazol, isoxazol, dihydroisoxazol, pyrazol and tetrazol,
where
R33 is hydrogen, 1-4C-alkyl, hydroxy-1-4C-alkyl, 1-4C-alkoxy, 2-4C-alkenyloxy, 1-4C-alkylcarbonyl, carboxy, 1-4C-alkoxycarbonyl, carboxy-1-4C-alkyl, 1-4C-alkoxycarbonyl-1-4C-alkyl, halogen, hydroxy, aryl, aryl-1-4C-alkyl, aryl-oxy, aryl-1-4C-alkoxy, trifluoromethyl, nitro, amino, mono- or di-1-4C-alkylamino, 1-4C-alkylcarbonylamino, 1-4Calkoxycarbonylamino, 1-4C-alkoxy-1-4C-alkoxycarbonylamino or sulfonyl,
R34 is hydrogen, 1-4C-alkyl, 1-4C-alkoxy, 1-4C-alkoxycarbonyl, halogen, trifluoromethyl or
hydroxy,
R35 is hydrogen, 1-4C-alkyl, 1-4C-alkoxy, 1-4C-alkoxycarbonyl, halogen, trifluoromethyl or hydroxy,
- R4: is hydrogen, halogen, 1-4C-alkyl, 3-7C-cycloalkyl, 3-7C-cycloalkyl-1-4C-alkyl, 14C-alkoxy-14C-alkyl, fluoro-1-4C-alkyl, fluoro-1-4C-alkoxy-1-4C-alkyl, hydroxy-1-4C-alkyl, hydroxy, 1-4C-alkoxy, 3-7C-cycloalkoxy, 3-7C-cycloalkyl-1-4C-alkoxy, 1-4C-alkoxy-1-4C-alkoxy, fluoro-1-4C-alkoxy, fluoro-1-4C-alkoxy-1-4C-alkoxy, hydroxy-1-4C-alkoxy, 2-4C-alkenyloxy or 2-4C-alkynyloxy
- R5: is hydrogen, 1-4C-alkyl, 3-7C-cycloalkyl, 3-7C-cycloalkyl-1-4C-alkyl, 1-4C-alkoxy-1-4C-alkyl, fluoro-1-4C-alkyl, fluoro-1-4C-alkoxy- 1-4C-alkyl or hydroxy-1-4C-alkyl,
- R6: is hydrogen, halogen, 1-4C-alkyl or fluoro-1-4C-alkyl,
- R7: is hydrogen, halogen, 1-4C-alkyl or fluoro-1-4C-alkyl,
and wherein
- aryl: is phenyl or substituted phenyl with one, two or three identical or different substituents from the group of 1-4C-alkyl, 1-4C-alkoxy, carboxy, 1-4C-alkoxycarbonyl, halogen, trifluoromethyl, nitro, trifluoromethoxy, hydroxy and cyano,
and the salts of these compounds.

Compounds of the formula 1a, which are to be mentioned are those, where
- R1: is hydrogen, 1-4C-alkyl or 3-7C-cycloalkyl,
- R2: is hydrogen or 1-4C-alkyl,
- R3: is halogen, fluoro-1-4C-alkyl, carboxyl, -CO-1-4C-alkoxy, hydroxy-1-4C-alkyl, 1-4C-alkoxy-1-4C-alkyl, 1-4C-alkoxy-1-4C-alkoxy-1-4C-alkyl, fluoro-1-4C-alkoxy-1-4C-alkyl or the group -CO-NR31R32,
where
R31 is hydrogen, hydroxyl, 1-7C-alkyl, hydroxy-1-4C-alkyl, 1-4C-alkoxy-1-4C-alkyl or 3-7C-cycloalkyl
and
R32 is hydrogen, 1-7C-alkyl, hydroxy-1-4C-alkyl or 1-4C-alkoxy-1-4C-alkyl,
or where
R31 and R32 together, including the nitrogen atom to which both are bonded, are a pyrrolidino, piperidino, piperazino, N-1-4C-alkylpiperazino, morpholino, aziridino or azetidino group,
- R4: is hydrogen, halogen, hydroxyl, 1-4C-alkyl, 3-5C-cycloalkyl, 1-4C-alkoxy, 3-5C-cycloalkoxy, 1-4C-alkoxy-1-4C-alkoxy, fluoro-1-4C-alkoxy, fluoro-1-4C-alkoxy-1-4C-alkoxy, hydroxy-1-4C-alkoxy, 2-4C-alkenyloxy or 2-4C-alkynyloxy and
- R5: is hydrogen, 1-4C-alkyl or hydroxy-1-4C-alkyl,
- R6: is hydrogen, fluoro, 1-4C-alkyl or fluoro-1-4C-alkyl,
- R7: is hydrogen, fluoro, 1-4C-alkyl or fluoro-1-4C-alkyl,
and the salts of these compounds.

Compounds of the formula 1a, which are to be particularly mentioned are those, where
- R1: is hydrogen or 1-4C-alkyl,
- R2: is 1-4C-alkyl,
- R3: is carboxyl, -CO-1-4C-alkoxy, hydroxy-1-4C-alkyl, 1-4C-alkoxy-1-4C-alkyl or the group -CO-NR31R32,
where
R31 is hydrogen, 1-7C-alkyl, hydroxy-1-4C-alkyl, or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy,
- R5: is hydrogen,
- R6: is hydrogen,
- R7: is hydrogen
and the salts of these compounds.

Compounds of the formula 1a, which are also to be particularly mentioned are those, where
- R1: is hydrogen or 1-4C-alkyl,
- R2: is 1-4C-alkyl,
- R3: is carboxyl, -CO-1-4C-alkoxy, hydroxy-1-4C-alkyl, 1-4C-alkoxy-1-4C-alkyl or the group -CO-NR31R32,
where
R31 is hydrogen, 1-7C-alkyl, hydroxy-1-4C-alkyl, or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy,
- R5: is hydrogen or 1-4C-alkyl,
- R6: is hydrogen,
- R7: is hydrogen
and the salts of these compounds.

Compounds of the formula 1a, which are to be emphasized are those, where
- R1: is hydrogen or 1-4C-alkyl,
- R2: is 1-4C-alkyl
- R3: is carboxyl, -CO-1-4C-alkoxy or the group -CO-NR31 R32,
where
R31 is hydrogen, 1-7C-alkyl, hydroxy-1-4C-alkyl, or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy,
- R5: is hydrogen,
- R6: is hydrogen,
- R7: is hydrogen
and the salts of these compounds.

Compounds of the formula 1a, which are also to be emphasized are those, where
- R1: is hydrogen or 1-4C-alkyl,
- R2: is 1-4C-alkyl
- R3: is carboxyl, -CO-1-4C-alkoxy or the group -CO-NR31R32,
where
R31 is hydrogen, 1-7C-alkyl, hydroxy-1-4C-alkyl, or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4Galkynyloxy,
- R5: is hydrogen or 1-4C-alkyl,
- R6: is hydrogen,
- R7: is hydrogen
and the salts of these compounds.

Compounds of the formula 1 a, which are further to be emphasized are those, where
- R1: is hydrogen or 1-4C-alkyl,
- R2: is 1-4C-alkyl,
- R3: is -CO-1-4C-alkoxy or the group -CO-NR31 R32,
where
R31 is hydrogen or 1-4C-alkyl,
R32 is hydrogen or 1-4C-alkyl,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy
- R5: is hydrogen,
- R6: is hydrogen,
- R7: is hydrogen
and the salts of these compounds.

Compounds of the formula 1a, which are also further to be emphasized are those, where
- R1: is hydrogen or 1-4C-alkyl,
- R2: is 1-4C-alkyl,
- R3: is -CO-1-4C-alkoxy or the group -CO-NR31 R32,
where
R31 is hydrogen or 1-4C-alkyl,
R32 is hydrogen or 1-4C-alkyl,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy
- R5: is hydrogen or 1-4C-alkyl,
- R6: is hydrogen,
- R7: is hydrogen
and the salts of these compounds.

Compounds of the formula 1a, which are to be particularly emphasized are those
in which
- R1: is 1-4C-alkyl,
- R2: is 1-4C-alkyl,
- R3: is -CO-N R31 R32,
where
R31 is hydrogen or 1-4C-alkyl
R32 is hydrogen or 1-4C-alkyl,
- R4: is hydrogen, hydroxyl, 1-4C-alkoxy, fluoro-1-4C-alkoxy or 1-4C-alkoxy-1-4C-alkoxy,
- R5: is hydrogen,
- R6: is hydrogen,
- R7: is hydrogen
and the salts of these compounds.

Compounds of the formula 1a, which are also to be particularly emphasized are those
in which
- R1: is 1-4C-alkyl,
- R2: is 1-4C-alkyl,
- R3: is -CO-NR31R32,
where
R31 is hydrogen or 1-4C-alkyl
R32 is hydrogen or 1-4C-alkyl,
- R4: is hydrogen, hydroxyl, 1-4C-alkoxy, fluoro-1-4C-alkoxy or 1-4C-alkoxy-1-4C-alkoxy,
- R5: is hydrogen or 1-4C-alkyl,
- R6: is hydrogen,
- R7: is hydrogen
and the salts of these compounds.

Another embodiment (embodiment b) of the invention to be emphasized are compounds of the formula 1b 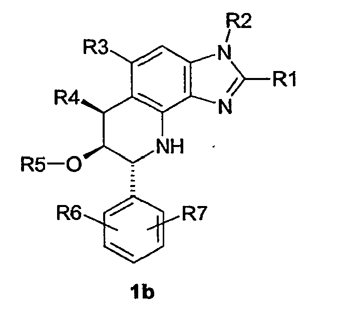 in which
- R1: is hydrogen, halogen, 1-4C-alkyl, 3-7C-cycloalkyl, 3-7C-cycloalkyl-1-4C-alkyl, 1-4C-alkoxy, 1-4C-alkoxy-1-4C-alkyl, 1-4C-alkoxycarbonyl, 2-4C-alkenyl, 2-4C-alkynyl, fluoro-1-4C-alkyl, fluoro-1-4C-alkoxy-1-4C-alkyl or hydroxy-1-4C-alkyl,
- R2: is hydrogen, 1-4C-alkyl, 3-7C-cycloalkyl, 3-7C-cycloalkyl-1-4C-alkyl, 1-4C-alkoxy- 1-4C-alkyl, 2-4C-alkenyl, 2-4C-alkynyl, fluoro-1-4C-alkyl or hydroxy-1-4C-alkyl,
- R3: is halogen, fluoro-1-4C-alkyl, carboxyl, -CO-1-4C-alkoxy, hydroxy-1-4C-alkyl, 1-4C-alkoxy-1-4C-alkyl, 1-4C-alkoxy-1-4C-alkoxy-1-4C-alkyl, fluoro-1-4C-alkoxy-1-4C-alkyl, cyano, the group -CO-NR31 R32, the group SO₂-NR31 R32 or the group Het,
where
R31 is hydrogen, hydroxyl, 1-7C-alkyl, hydroxy-1-4C-alkyl, 1-4C-alkoxy-1-4C-alkyl, 3-7C-cycloalkyl or amino and
R32 is hydrogen, 1-7C-alkyl, hydroxy-1-4C-alkyl or 1-4C-alkoxy-1-4C-alkyl,
or where
R31 and R32 together, including the nitrogen atom to which both are bonded, are a pyrrolidino, piperidino, piperazino, N-1-4C-alkylpiperazino, morpholino, aziridino or azetidino group and Het is a heterocyclic residue, substituted by R33, R34 and R35, selected from the group consisting of oxadiazol, dihydrooxazol, dihydroimidazol, oxazol, imidazol, isoxazol, dihydroisoxazol, pyrazol and tetrazol,
where
R33 is hydrogen, 1-4C-alkyl, hydroxy-1-4C-alkyl, 1-4C-alkoxy, 2-4C-alkenyloxy, 1-4C-alkylcarbonyl, carboxy, 1-4C-alkoxycarbonyl, carboxy-1-4C-alkyl, 1-4C-alkoxycarbonyl-1-4Calkyl, halogen, hydroxy, aryl, aryl-1-4C-alkyl, aryi-oxy, aryl-1-4C-alkoxy, trifluoromethyl, nitro, amino, mono- or di-1-4C-alkylamino, 1-4C-alkylcarbonylamino, 1-4Calkoxycarbonylamino, 1-4C-alkoxy-1-4C-alkoxycarbonylamino or sulfonyl,
R34 is hydrogen, 1-4C-alkyl, 1-4C-atkoxy, 1-4C-alkoxycarbonyl, halogen, trifluoromethyl or hydroxy,
R35 is hydrogen, 1-4C-alkyl, 1-4C-alkoxy, 1-4C-alkoxycarbonyl, halogen, trifluoromethyl or hydroxy,
- R4: is hydrogen, halogen, 1-4C-alkyl, 3-7C-cycloalkyl, 3-7C-cycloalkyl-1-4C-alkyl, 1-4C-alkoxy-1-4C-alkyl, fluoro-1-4C-alkyl, fluoro-1-4C-alkoxy-1-4C-alkyl, hydroxy-1-4C-alkyl, hydroxy, 1-4C-alkoxy, 3-7C-cycloalkoxy, 3-7C-cycloalkyl-1-4C-alkoxy, 1-4C-alkoxy-1-4C-alkoxy, fluoro-1-4C-alkoxy, fluoro-1-4C-alkoxy-1-4C-alkoxy, hydroxy-1 -4C-alkoxy, 2-4C-alkenyloxy or 2-4C-alkynyloxy
- R5: is hydrogen, 1-4C-alkyl, 3-7C-cycloalkyl, 3-7C-cycloalkyl-1-4C-alkyl, 1-4C-alkoxy-1-4C-alkyl, fluoro-1-4C-alkyl, fluoro-1-4C-alkoxy- 1-4C-alkyl or hydroxy-1-4C-alkyl,
- R6: is hydrogen, halogen, 1-4C-alkyl or fluoro-1 -4C-alkyl,
- R7: is hydrogen, halogen, 1-4C-alkyl or fluoro-1-4C-alkyl,
and wherein
- aryl: is phenyl or substituted phenyl with one, two or three identical or different substituents from the group of 1-4C-alkyl, 1-4C-alkoxy, carboxy, 1-4C-alkoxycarbonyl, halogen, trifluoromethyl, nitro, trifluoromethoxy, hydroxy and cyano,
and the salts of these compounds.

Compounds of the formula 1b, which are to be mentioned are those, where
- R1: is hydrogen, 1-4C-alkyl or 3-7C-cycloalkyl,
- R2: is hydrogen or 1-4C-alkyl,
- R3: is halogen, fluoro-1-4C-alkyl, carboxyl, -CO-1-4C-alkoxy, hydroxy-1-4C-alkyl, 1-4C-alkoxy-1-4C-alkyl, 1-4C-alkoxy-1-4C-alkoxy-1-4C-alkyl, fluoro-1-4C-alkoxy-1-4C-alkyl or the group -CO-NR31 R32,
where
R31 is hydrogen, hydroxyl, 1-7C-alkyl, hydroxy-1 -4C-alkyl, 1-4C-alkoxy-1-4C-alkyl or 3-7C-cycloalkyl
and
R32 is hydrogen, 1-7C-alkyl, hydroxy-1-4C-alkyl or 1-4C-alkoxy-1-4C-alkyl,
or where
R31 and R32 together, including the nitrogen atom to which both are bonded, are a pyrrolidino, piperidino, piperazino, N-1-4C-alkylpiperazino, morpholino, aziridino or azetidino group,
- R4: is hydrogen, halogen, hydroxyl, 1-4C-alkyl, 3-5C-cycloalkyl, 1-4C-alkoxy, 3-5C-cycloalkoxy, 1-4C-alkoxy-1-4C-alkoxy, fluoro-1-4C-alkoxy, fluoro-1-4C-alkoxy-1-4C-alkoxy, hydroxy-1-4C-alkoxy, 2-4C-alkenyloxy or 2-4C-alkynyloxy and
- R5: is hydrogen, 1-4C-alkyl or hydroxy-1-4C-alkyl,
- R6: is hydrogen, fluoro, 1-4C-alkyl or fluoro-1-4C-alkyl,
- R7: is hydrogen, fluoro, 1-4C-alkyl or fluoro-1-4C-alkyl,
and the salts of these compounds.

Compounds of the formula 1b, which are to be particularly mentioned are those, where
- R1: is hydrogen or 1-4C-alkyl,
- R2: is 1-4C-alkyl,
- R3: is carboxyl, -CO-1-4C-alkoxy, hydroxy-1-4C-alkyl, 1-4C-alkoxy-1-4C-alkyl or the group -CO-NR31 R32,
where
R31 is hydrogen, 1-7C-alkyl, hydroxy-1-4C-alkyl, or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy,
- R5: is hydrogen,
- R6: is hydrogen,
- R7: is hydrogen
and the salts of these compounds.

Compounds of the formula 1b, which are also to be particularly mentioned are those, where
- R1: is hydrogen or 1-4C-alkyl,
- R2: is 1-4C-alkyl,
- R3: is carboxyl, -CO-1-4C-alkoxy, hydroxy-1-4C-alkyl, 1-4C-alkoxy-1 -4C-alkyl or the group -CO-NR31 R32,
where
R31 is hydrogen, 1-7C-alkyl, hydroxy-1-4C-alkyl, or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy,
- R5: is hydrogen or 1-4C-alkyl,
- R6: is hydrogen,
- R7: is hydrogen
and the salts of these compounds.

Compounds of the formula 1b, which are to be emphasized are those, where
- R1: is hydrogen or 1-4C-alkyl,
- R2: is 1-4C-alkyl
- R3: is carboxyl, -CO-1-4C-alkoxy or the group -CO-NR31R32,
where
R31 is hydrogen, 1-7C-alkyl, hydroxy-1-4C-alkyl, or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy,
- R5: is hydrogen,
- R6: is hydrogen,
- R7: is hydrogen
and the salts of these compounds.

Compounds of the formula 1b, which are also to be emphasized are those, where
- R1: is hydrogen or 1-4C-alkyl,
- R2: is 1-4C-alkyl
- R3: is carboxyl, -CO-1-4C-alkoxy or the group -CO-NR31 R32,
where
R31 is hydrogen, 1-7C-alkyl, hydroxy-1-4C-alkyl, or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy,
- R5: is hydrogen or 1-4C-alkyl,
- R6: is hydrogen,
- R7: is hydrogen
and the salts of these compounds.

Compounds of the formula 1b, which are further to be emphasized are those, where
- R1: is hydrogen or 1-4C-alkyl,
- R2: is 1-4C-alkyl,
- R3: is -CO-1-4C-alkoxy or the group -CO-NR31 R32,
where
R31 is hydrogen or 1-4C-alkyl,
R32 is hydrogen or 1-4C-alkyl,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy
- R5: is hydrogen,
- R6: is hydrogen,
- R7: is hydrogen
and the salts of these compounds.

Compounds of the formula 1b, which are also further to be emphasized are those, where
- R1: is hydrogen or 1-4C-alkyl,
- R2: is 1-4C-alkyl,
- R3: is -CO-1-4C-alkoxy or the group -CO-NR31R32,
where
R31 is hydrogen or 1-4C-alkyl,
R32 is hydrogen or 1-4C-alkyl,
- R4: is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy
- R5: is hydrogen or 1-4C-alkyl,
- R6: is hydrogen,
- R7: is hydrogen
and the salts of these compounds.

Compounds of the formula 1b, which are to be particularly emphasized are those
in which
- R1: is 1-4C-alkyl,
- R2: is 1-4C-alkyl,
- R3: is -CO-NR31 R32,
where
R31 is hydrogen or 1-4C-alkyl
R32 is hydrogen or 1-4C-alkyl,
- R4: is hydrogen, hydroxyl, 1-4C-alkoxy, fluoro-1-4C-alkoxy or 1-4C-alkoxy-1-4C-alkoxy,
- R5: is hydrogen,
- R6: is hydrogen,
- R7: is hydrogen
and the salts of these compounds.

Compounds of the formula 1b, which are also to be particularly emphasized are those
in which
- R1: is 1-4C-alkyl,
- R2: is 1-4C-alkyl,
- R3: is -CO-NR31R32,
where
R31 is hydrogen or 1-4C-alkyl
R32 is hydrogen or 1-4C-alkyl,
- R4: is hydrogen, hydroxyl, 1-4C-alkoxy, fluoro-1-4C-alkoxy or 1-4C-alkoxy-1-4C-alkoxy,
- R5: is hydrogen or 1-4C-alkyl,
- R6: is hydrogen,
- R7: is hydrogen
and the salts of these compounds.

Particularly preferred are the compounds given as final products of formula 1 in the examples, and the
salts of these compounds.

The compounds according to the invention can be synthesized from corresponding starting compounds, for example according to the reaction schemes given below. The synthesis is carried out in a manner known to the expert, for example as described in more detail in the following examples.

The compounds of the formula 1 can be obtained for example starting from compounds of the formula 2 following the reaction sequences shown in scheme 1 or shown in scheme 2. Oxidation of compounds of the formula 2 to compounds of the formula 3 is performed by standard procedures, for example using quinones (e.g. chloranil or DDQ).

Scheme 1 shows that, after deprotection of compounds of the formula 3, the reduction of the keto group (e.g. using sodium boronhydride) delivers the corresponding diols of the formula 1 with R4 = OH and R5 = H, followed, if desired, by customary derivatization reactions which are familiar to the person skilled in the art (e.g. by alkylation) to give compounds of the formula 1 with for example R4 = 1-4C-alkoxy, 1-4C-alkoxy-1-4C-alkoxy, or fluoro-1-4C-alkoxy.

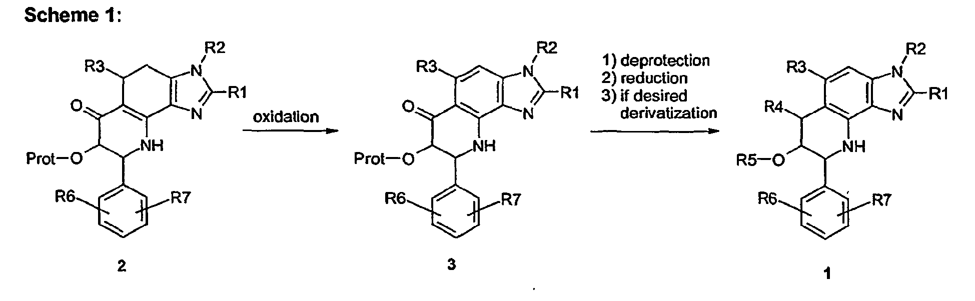

Scheme 2 illustrates the derivatization of the keto group of compounds of the formula 3 by reactions, which are familiar to the person skilled in the art (e.g. by C-C-bond forming reactions like the Wittig reaction followed by hydrogenation or by reduction followed by transforming of the resulted hydroxyl group into a halogen group). Deprotection and, if desired, further customary derivatization reactions in manner known to the expert (e.g. by alkylation) give compounds of the formula 1 with for example R4 = halogen, 1-4C-alkyl, 3-7C-cycloalkyl, 1-4C-alkoxy-1-4C-alkyl, or fluoro-1-4C-alkyl.

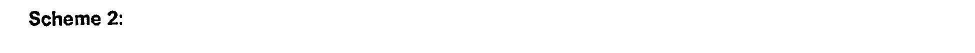

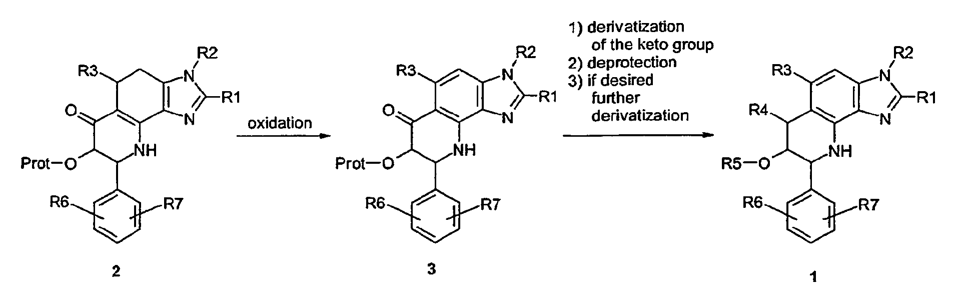

In the case of compounds of the formula 1 with R4 = OH and R5 = H further derivatizations are possible, for example as shown in scheme 3. Formation of compounds of the formula 4 by methodologies well known to the experts, for example under Mitsunobu-conditions, followed by opening of the epoxide ring by nucleophiles and, if desired, customary derivatization reactions delivers further compounds of the formula 1 with for example R4 = hydrogen, 1-4C-alkyl, 3-7C-cycloalkyl, 1-4C-alkoxy-1-4C-alkyl, or fluoro-1-4C-alkyl.

Depending on the conditions of the epoxide formation and the derivatization reactions, respectively, the free amino-functionality in compounds of the formula 1 with R4 = OH and R5 = H and/or in compounds of the formula 4 has to be protected with a suitable protection group, for example the acetyl group.

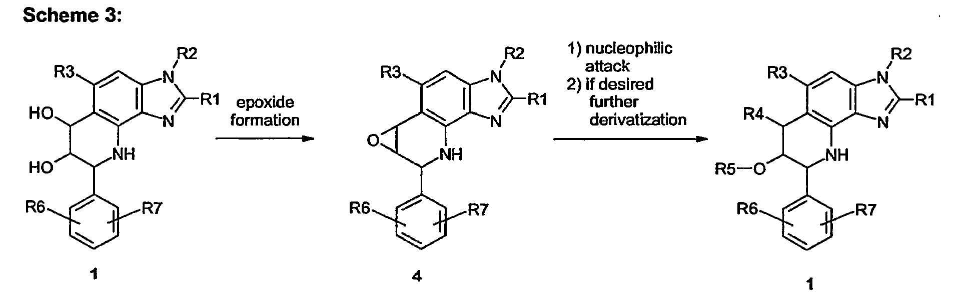

Compounds of the formula 2 can be prepared for example as outlined in scheme 4. In a first step ketones of the formula 5 are reacted with protected phenylisoserine derivatives of the formula 6 (wherein Y is a suitable leaving group, for example an ethoxy group and Prot is a suitable protecting group like a suitable silyl radical, for example a ^{t}BuMe₂Si-radical) to give compounds of the formula 2 and/or compounds of the formula 7. Compounds of the formula 7, if obtained, can be re-protected by standard procedures to the desired compounds of the formula 2.

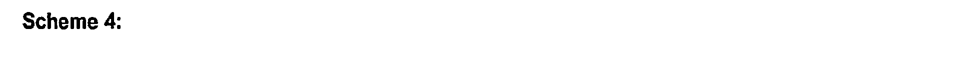

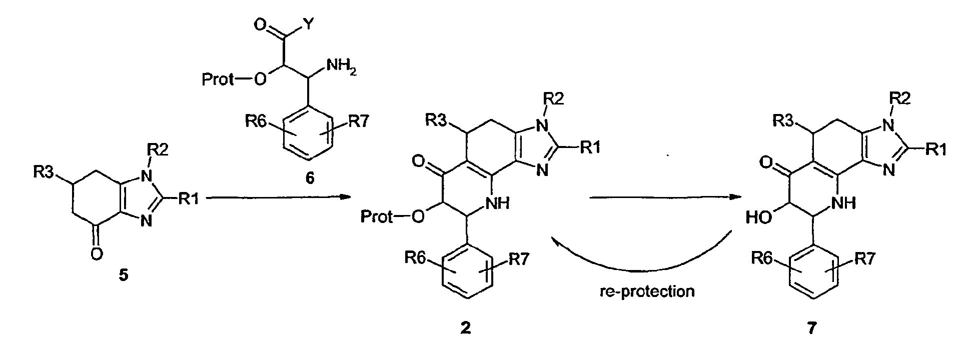

Compounds of the formula 2, initially obtained with a silyl protecting group, are preferably separated from or transformed to compounds of the formula 7. Compounds of the formula 7 can be re-protected by standard procedures to the desired compounds of the formula 2, wherein the protecting group is then preferably an acetyl or a pivaloyl group, which is more stable in the further course of the reaction as compared to the initially obtained silyl protecting group.

The synthesis as outlined in scheme 4a leads to the preferred optically pure compounds of the formula 1a and of the formula 1 b by reacting ketones of the formula 5 with optically pure phenylisoserine derivatives of the formula 6a and further chemical transformations as described in scheme 1, scheme 2, and scheme 3. The configuration of the substituent R4 forming either compounds of the formula 1a or compounds of the formula 1b can be controlled by the choice of the derivatization reactions.

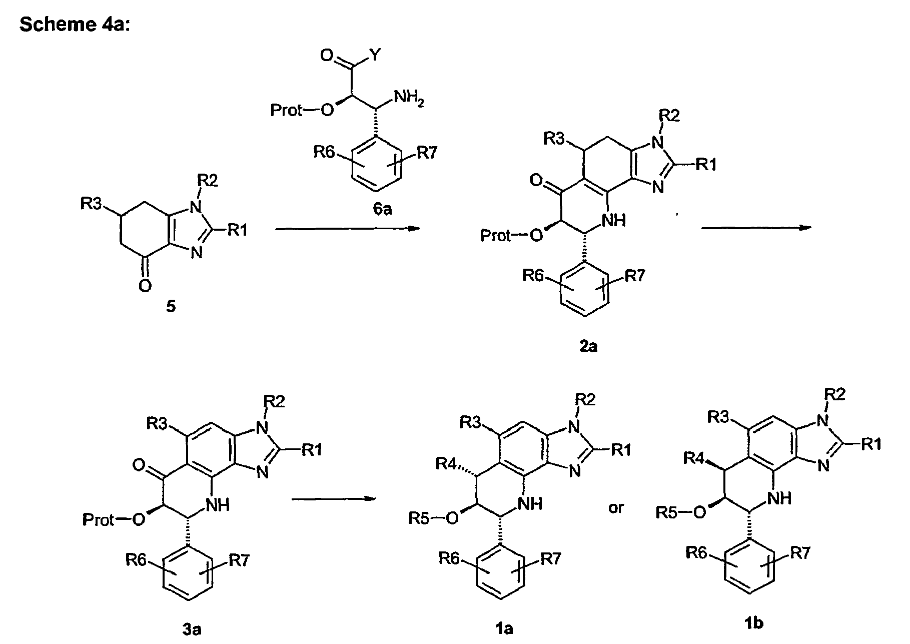

Ketones of the formula 5 can be prepared as shown, for example, in scheme 5 (route A) performing the cyclization reaction of compounds of the formula 9 in the presence of a primary amine (R2 ≠H) or ammonia (R2 = H). The preparation of compounds of the formula 9 can be achieved by several methodologies known to the expert; two examples are illustrated in scheme 5 (route A): The reduction of azo-compounds of the formula 8 by methodologies which are performed in manner known to the expert, for example as described by A. Treibs, R. Zinsmeister in Chem. Ber. (1957), 90,87-92, followed by the acylation delivers compounds of formula 9. Alternatively, aromatic compounds of the formula 10 can be reduced by strong reducing agents followed by an acidic workup, for example as described by Kuehne, Lambert in Org. Synth.; Coll. Vol. V, (1973), 400 or by A. Mann, C. Humblet in J. Med. Chem., (1985), 28, 1440-1446. Compounds of the formula 8 and 10 are known, for example from the French Patent FR2242984*,* or from Allan, Collect. Czech. Chem. Commun. (1966), 31, 4129, or they can be prepared using analogues process steps.

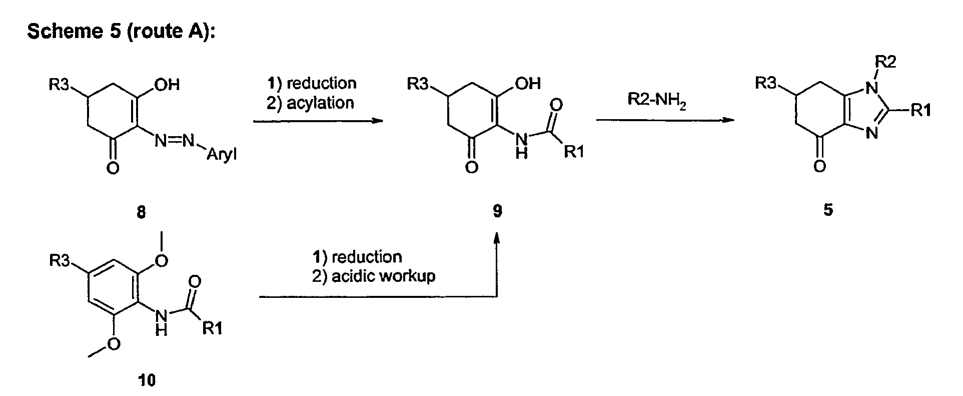

Alternatively, the ketones of the formula 5 can be prepared in a manner as shown for example in scheme 6 (route B). Compounds of the formula 11 can be hydrogenated to the desired compounds of the formula 5 in manner known to the expert, for example as described by H. Oelschlaeger and H. Giebenhain in Archiv der Pharmazie, 1973, 306, 485-489. The starting compounds 11 are known, for example, from A.R. Katritzky et al., Heterocycles (1995), 41, 345-352, or they can be prepared using analogous process steps.

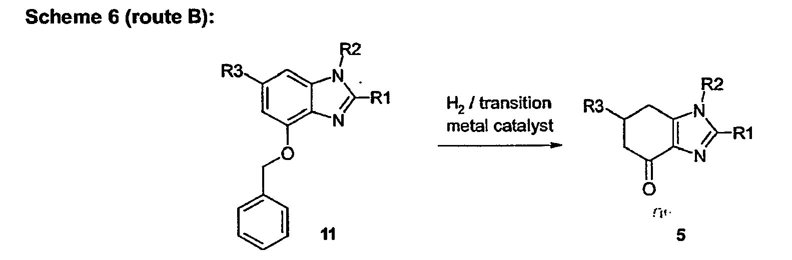

Phenylisoserine derivatives of the formula 6 or 6a can be prepared in analogy to methods known in literature (see for example J. Amer. Chem. Soc. (1998), 120, 431) or by methods known to the expert, for example by reaction under basic conditions of the corresponding unprotected phenylisoserine derivatives of the formula 12 with suitable protection group precursor Prot-Z with a suitable leaving group Z, like a suitable silyl chloride, for example ^{t}BuMe₂SiCl, as shown in Scheme 7.

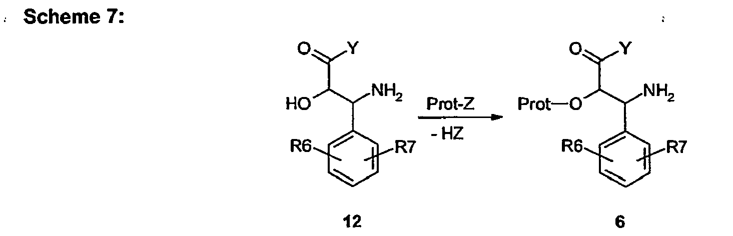

Compounds of the formula 12 are known or can be prepared by methods known to the expert, for example by epoxidation of the corresponding cinnamic acid derivatives of the formula 13, followed by a ring opening reaction or directly by a aminohydroxylation reaction. Both variants can be performed in a stereoselective way, which leads for example to compounds of the formula 12a, as shown in Scheme 7a.

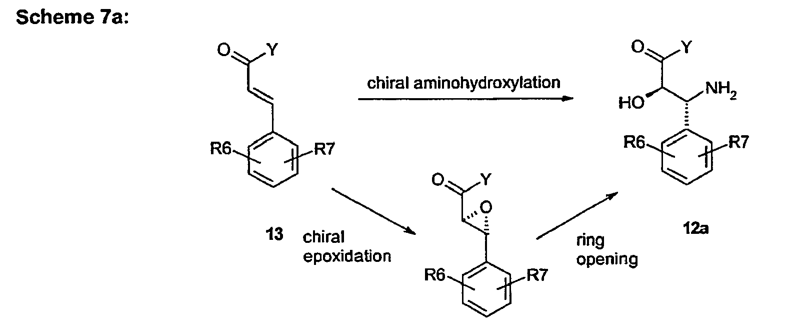

The derivatization, if any, of the compounds obtained according to the schemes above (e.g. conversion of a group R3 into another group R3 or conversion of a hydroxyl group into an alkoxy or ester group) is likewise carried out in a manner known per se. If, for example, compounds of the formula 1 or 1 a where R3 = -CO-NR31 R32 are desired, an appropriate derivatization can be performed in a manner known per se (e. g. conversion of an ester or a carboxylic acid into an amide), for example at the stage of compounds of the formula 1.

The reaction steps outlined above are carried out in a manner known per se, e.g. as described in more detail in the examples.

The following examples serve to illustrate the invention in greater detail without restricting it. Likewise, further compounds of the formula 1 whose preparation is not described explicitly can be prepared in an analogous manner or in a manner familiar per se to the person skilled in the art using customary process techniques. The abbreviation min stands for minute(s), h for hour(s), m.p. for melting point, MS for mass spectrometry and ESI for electro spray ionization.

### Examples

### I. Final Products of formula 1

### 1. (65,7R,8R)-[6,7,8,9-Tetrahydro-6,7-dihydroxy-2,3-dimethyl-8-phenyl-3H-imidazo[4,5-h]quinoline-5-yl]-carboxylic acid methylamide

To a at 0°C cooled stirred solution of 0.30 g (0.82 mmol) (7R,8R)-[6,7,8,9-tetrahydro-7-hydroxy-2,3-dimethyl-6-oxo-8-phenyl-3*H*-imidazo[4,5-h]quinoline-5-yl]-carboxylic acid methylamide in 10 ml methanol were added 0.062 g (1.64 mmol) sodium borohydride. The reaction mixture was stirred for 1h at 0°C and 3 h at room temperature. The solution was concentrated, the residue poured onto a saturated sodium chloride solution, and extracted with chloroform five times. The combined organic layers were washed with a small amount of water, dried (MgSO₄), concentrated in vacuo, and purified by flash column chromatography (silica gel, dichloromethane/methanol 100 / 3). Crystallization from diisopropyl ether gave 0.035 g (12 %) of the title product as white crystals.
¹H-NMR (200MHz, CDCl₃): δ [ppm] = 2.51 (s, 3 H), 3.38 (s, 3 H), 3.54-3.63 (m, 2 H), 3.66 (s, 3 H), 3.86-4.11 (m, 2 H), 4.21 (dd, 1H), 4.49 (d, 1H), 4.87 (dd, 1H), 6.67 (d, 1H), 7.23 (1d, 1H), 7.31-7.42 (m, 3 H), 7.52-7.56 (m, 2H).

### 2. (6R,7R,8R)-[6,7,8,9-Tetrahydro-6,7-dihydroxy-2,3-dimethyl-8-phenyl-3H-imidazo[4,5-h]quinoline-5-yl]-carboxylic acid methylamide

To a at 0°C cooled stirred solution of 0.30 g (0.82 mmol) (7R,8R)-[6,7,8,9-tetrahydro-7-hydroxy-2,3-dimethyl-6-oxo-8-phenyl-3*H*-imidazo[4,5-h]quinoline-5-yl]-carboxylic acid methylamide in 10 ml methanol were added 0.062 g (1.64 mmol) sodium borohydride. The reaction mixture was stirred for 1 h at 0°C and 3 h at room temperature. The solution was concentrated, the residue poured onto a saturated sodium chloride solution, and extracted with chloroform five times. The combined organic layers were washed with a small amount of water, dried (MgSO₄), concentrated in vacuo, and purified by flash column chromatography (silica gel, dichloromethane/methanol 100 /3). Crystallization from diisopropyl ether gave 0.197 g (65 %) of the title product as white crystals.
¹H-NMR (200MHz. CDCl₃): δ [ppm] = 2.51 (s, 3 H), 3.38 (s, 3 H), 3.54-3.63 (m, 2 H), 3.66 (s, 3 H), 3.86-4.11 (m, 2 H), 4.21 (dd, 1H), 4.49 (d, 1H), 4.87 (dd, 1H), 6.67 (d, 1H), 7.23 (1d, 1H), 7.31-7.42 (m, 3 H), 7.52-7.56 (m, 2 H).

### 3. (6S,7R,8R)-[6-n-butyloxy-6,7,8,9-tetrahydro-7-hydroxy-2,3-dimethyl-8-phenyl-3H-imidazo[4,5-h]quinoline-5-yl]-carboxylic acid methylamide

To a at-30°C cooled stirred solution of 0.50 g (1.36 mmol) (6R,7R,8R)-[6,7,8,9-tetrahydro-6,7-dihydroxy-2,3-dimethyl-8-phenyl-3*H*-imidazo[4,5-h]quinoline-5-yl]-carboxylic acid in 10 ml n-butanol were added dropwise 0.12 ml (1.77 mmol) methansulfonic acid. The reaction mixture was stirred for 30 min at -30°C and was then warmed to room temperature. The solution was poured onto water, neutralized with a saturated sodium hydrogen carbonate solution, and extracted with dichloromethane. The combined organic layers were washed with water, dried (MgSO₄), concentrated in vacuo, and purified by flash column chromatography (silica gel, dichloromethane/methanol 100 / 3) to give, after crystallization from diisopropyl ether, 0.258 g (45 %) of the title product as white crystals.
¹H-NMR (200MHz, d₆-DMSO): δ [ppm] = 0.85 (t, 3H), 1.20-1.52 (m, 4H), 2.48 (s, 3H), 2.78 (d, 3H), 3.40-3.51 (m, 1H), 3.57-3.67 (m, 4H), 3.79-3.89 (m, 1H). 4.46 (d, 1H), 4.61 (d, 1H). 5.00 (d, 1H), 5.43 (s, 1H), 6.75 (s, 1H), 7.25-7.44 (m, 5H), 8.08 (d, 1H).

### 4. (6R,7R,8R)-[6-n-butyloxy-6,7,8,9-tetrahydro-7-hydroxy-2,3-dimethyl-8-phenyl-3H-imidazo[4,5-h]quinoline-5-yl]-carboxylic acid methylamide

To a at-30°C cooled stirred solution of 0.50 g (1.36 mmol) (6R,7R,8R)-[6,7,8,9-tetrahydro-6,7-dihydroxy-2,3-dimethyl-8-phenyl-3*H*-imidazo[4,5-h]quinoline-5-yl]-carboxylic acid methylamide in 10 ml *n*-butanol were added dropwise 0.12 ml (1.77 mmol) methansulfonic acid. The reaction mixture was stirred for 30 min at -30°C and was then warmed to room temperature. The solution was poured onto water, neutralized with a saturated sodium hydrogen carbonate solution, and extracted with dichloromethane. The combined organic layers were washed with water, dried (MgSO₄), concentrated in vacuo and purified by flash column chromatography (silica gel, dichloromethane/methanol 100 / 3) to give, after crystallization from diisopropyl ether, 0.175 g (30 %) of the title product as white crystals.
¹H-NMR (200MHz, d₆-DMSO): δ [ppm] = 0.67 (t, 3H), 0.82-1.05 (m, 4H), 2.52 (s, 3H), 2.71 (d, 3H), 3.12-3.31 (m, 5H), 3.68 (s, 3H), 4.18 (dd, 1H), 4.47 (t, 1H), 4.78 (d, 1H). 4.85 (d, 1H), 5.90 (d, 1H), 6.76 (s, 1H). 7.12-7.37 (m, 5H), 7.80 (d, 1H).

### 5. (6S,7R,8R)-[6,7,8,9 Tetrahydro-7hydroxy-6-(2-methoxyethoxy)-2,3-dimethyl-8-phenyl-3H-imidazo[4,5-h]quinoline-5-yl]-carboxylic acid methylamide

To a at -30°C cooled stirred solution of 0.50 g (1.36 mmol) (6R,7R,8R)-[6,7,8.9-tetrahydro-6,7-dihydroxy-2,3-dimethyl-8-phenyl-3H-imidazo[4,5-h]quinoline-5-yl]-carboxylic acid methylamide in 10 ml 2-methoxyethanol were added dropwise 0.12 ml (1.77 mmol) methansulfonic acid. The solution was stirred for 1 hat -30°C and was then warmed to room temperature. Triethylamine (0.60 ml) was added and the reaction mixture was concentrated. The white crystalline residue was purified by flash column chromatography (silica gel, dichloromethane/methanol 100 / 3) to give, after crystallization from diisopropyl ether, 0.258 g (45 %) of the title product as white crystals.
¹H-NMR (200MHz, d₆-DMSO): δ [ppm] = 2.46 (s, 3H), 2.78 (d, 3H), 3.24 (s, 3H), 3.40-3.46 (m, 2H), 3.57-3.70 (m, 3H), 3.80-3.90 (m, 2H), 4.44 (d, 1H), 4.68 (d, 1H), 4.96 (d, 1H), 5.46 (s, 1H), 6.78 (s, 1 H), 7.29-7.45 (m, 5H), 8.01 (d, 1H).

### 6. (6R,7R,8R)-[6,7,8,9 Tetrahydro-7-hydroxy-6-(2-methoxyethoxy)-2,3-dimethyl-8-phenyl-3H-imidazo[4,5-h]quinoline-5-yl]-carboxylic acid methylamide

To a at -30°C cooled stirred solution of 0.50 g (1.36 mmol) (6R,7R,8R)-[6,7,8,9-tetrahydro-6,7-dihydroxy-2,3-dimethyl-8-phenyl-3*H*-imidazo[4,5-h]quinoline-5-yl]-carboxylic acid methylamide in 10 ml 2-methoxyethanol were added dropwise 0.12 ml (1.77 mmol) methansulfonic acid. The solution was stirred for 1 h at -30°C and was then warmed to room temperature. Triethylamine (0.60 ml) was added and the reaction mixture was concentrated. The white crystalline residue was purified by flash column chromatography (silica gel, dichloromethane/methanol 100 / 3) to give, after crystallization from diisopropyl ether, 0.049 g (9 %) of the title product as white crystals.
¹H-NMR (200MHz, d₆-DMSO): δ [ppm] = 2.46 (s, 3H), 2.72-2.81 (m, 4H), 2.95-3.03 (m, 1H), 3.06 (s, 3H), 3.17-3.30 (m, 2H), 3.68 (s, 3H), 4.23 (dd, 1H), 4.51 (t, 1H), 4.65 (d, 1H), 5.05 (d, 1H), 6.02 (d, 1H), 6.81 (s, 1H), 7.18-7.37 (m, 5H), 7.69 (d, 1H).

### 7. (6S,7R,8R)-[6,7,8,9-Tetrahydro-7-hydroxy-2,3-dimethyl-8-phenyl-6-propargyloxy-3H-imidazo[4,5-h]quinoline-5-yl]-carboxylic acid methylamide

To a at -30°C cooled stirred solution of 0.75 g (2.04 mmol) (6R,7R,8R)-[6,7,8,9-tetrahydro-6,7-dihydroxy-2,3-dimethyl-8-phenyl-3*H*-imidazo[4,5-h]quinoline-5-yl]-carboxylic acid methylamide in 5 ml propargylic alcohol were added dropwise 0.18 ml (2.66 mmol) methansulfonic acid. The solution was stirred for 1 h at -30°C and was then warmed to room temperature. The solution was poured onto water, neutralized with a sodium hydroxide solution (2N), and extracted with dichloromethane. The combined organic layers were washed with water, dried (MgSO₄), concentrated in vacuo and purified by flash column chromatography (silica gel, dichloromethane/methanol 100/3) to give, after crystallization from diisopropyl ether, 0.251 g (30 %) of the title product as white crystals.
1H-NMR (200MHz, d₆-DMSO): δ [ppm] = 2.47 (s, 3H), 2.78 (d, 3H), 3.25-3:32 (m, 1H), 3.67 (s, 3H), 3.82-3.91 (m, 1H), 4.18 (dd, 1H), 4.29 (dd, 1H), 4.46 (d, 1H), 4.86 (d, 1H), 5.05 (d, 1H), 5.52 (s, 1H), 6.77 (s, 1H), 7.29-7.44 (m, 5H), 8.12 (d, 1H).

### 8. (6R,7R,8R)-[6,7,8,9-Tetrahydro-7-hydroxy-2,3-dimethyl-8-phenyl-6-propargyloxy-3H-imidazo[4,5-h]quinoline-5-yl]-carboxylic acid methylamide

To a at-30°C cooled stirred solution of 0.75 g (2.04 mmol) (6R,7R,8R)-[6,7,8,9-tetrahydro-6,7-dihydroxy-2,3-dimethyl-8-phenyl-3*H*-imidazo[4,5-h]quinoline-5-yl]-carboxylic acid methylamide in 5 ml propargylic alcohol were added dropwise 0.18 ml (2.66 mmol) methansulfonic acid. The solution was stirred for 1 h at -30°C and was then warmed to room temperature. The solution was poured onto water, neutralized with a sodium hydroxide solution (2N), and extracted with dichloromethane. The combined organic layers were washed with water, dried (MgSO₄), concentrated in vacuo and purified by flash column chromatography (silica gel, dichloromethane/methanol 100/3) to give, after crystallization from diisopropyl ether, 0.150 g (18 %) of the title product as white crystals.
1H-NMR (200MHz, d₆-DMSO): δ [ppm] = 2.49 (s, 3H), 2.76 (d, 3H), 3.20-3.22 (m, 1H), 3.67 (s, 3H), 3.92 (dd, 1H), 4.03-4.22 (m, 2H), 4.35 (d, 1H), 4.90 (d, 1H), 5.10 (d, 1H), 5.61 (d, 1H), 6.80 (s, 1H), 7.20-7.46 (m, 5H), 7.87 (d, 1H).

### 9. (6S,7R,8R)-[6,7,8,9-Tetrahydro-7-hydroxy-2,3-dimethyl-8-phenyl-6-(2,2,2-trifluoroethyl)-3H-imidazo[4,5-h]quinoline-5-yl]-carboxylic acid methylamide

To a at -30°C cooled stirred solution of 0.75 g (2.66 mmol) (6R,7R,8R)-[6,7,8,9-tetrahydro-6,7-dihydroxy-2,3-dimethyl-8-phenyl-3*H*-imidazo[4,5-h]quinoline-5-yl]-carboxylic acid methylamide in 7.5 ml 2,2,2-trifluoroethanol were added dropwise 0.18 ml (1.77 mmol) methansulfonic acid. The solution was stirred for 1 h at -,30°C and was then warmed to room temperature. The solution was poured onto water, neutralized with a saturated sodium hydrogen carbonate solution, and extracted with dichloromethane. The combined organic layers were washed with water, dried (MgS0₄), concentrated in vacuo and purified by flash column chromatography (silica gel, dichloromethane/methanol 100 / 3) to give, after crystallization from diisopropyl ether/petroleum ether, 0.052 g (6 %) of the title product as white crystals.
¹H-NMR (200MHz, d₆-DMSO): δ [ppm] = 2.47 (s, 3H), 2.76 (d, 3H), 3.68 (s, 3H), 3.88-3.96 (m, 1H), 4.01-4.17 (m, 1H), 4.30-4.48 (m, 2H), 5.04 (d, 1H), 5.26 (d, 1H), 5.56 (s, 1H), 6.84 (s, 1H), 7.30-7.45 (m, 5H), 8.15 (d, 1H).

### 10. (6R,7R,8R)-[7-Hydroxy-2,3-dimethyl-8-phenyl-6-(2,2-difluoroethyl)-6,7,8,9-tetrahydro-3H-imidazo[4,5-h]quinoline-5-yl]-carboxylic acid methylamide

To a at -30°C cooled stirred solution of (6R,7R,8R)-[6,7-dihydroxy-2,3-dimethyl-8-phenyl-6,7,8,9-tetrahydro-3*H*-imidazo[4,5-h]quinoline-5-yl]-carboxylic acid methylamide (0.75 g, 2.04 mmol) in a mixture of 2,2-difluoroethanol (5 ml) and dichloromethane (3 ml) was added dropwise methansulfonic acid (0.26 ml, 2.66 mmol). The solution was stirred for 1 h at -30°C and was then warmed to room temperature. The solution was poured onto water, neutralized with a saturated sodium hydrogen carbonate solution, and extracted with dichloromethane. The combined organic layers were dried (MgSO₄), concentrated in vacuo and purified by flash column chromatography (silica gel, dichloromethane/methanol 100 / 3) to give, after crystallization from diisopropyl ether/petroleum ether, 0.18 g (21 %) of the title product as white crystals.
¹H-NMR (200MHz, d₆-DMSO): δ [ppm] = 2.47 (s, 3H), 2.77 (d, 3H), 3.67 (s, 3H), 3.72-.4.14 (m, 3H), 4.45 (d, 1H), 4.94 (d, 1H), 5.11 (s, 1H), 5.52 (s, 1H), 6.06 (tt, 1H), 6.82 (s, 1H), 7.23-7.45 (m, 5H), 8.13 (d, 1H).

### 11. (7R,8R)-[7-Hydroxy-2,3-dimethyl-8-phenyl-6,7,8,9-tetrahydro-3H-imidazo[4,5-h]quinoline-5-yl]-carboxylic acid methylamide

To a suspension of (6R,7R,8R)-[6,7-dihydroxy-2,3-dimethyl-8-phenyl-6,7,8,9-tetrahydro-3*H-*imidazo[4,5-h]quinoline-5-yl]-carboxylic acid methylamide (0.50 g, 1.36 mmol) in dichloromethane (10 ml) was added trifluoroacetic acid (1.20 ml). A clear solution resulted, triethylsilane (3.5 ml) was added, and the solution was stirred for 2 h at room temperature. The solution was poured onto water, neutralized with a sodium hydroxide solution (2N) (pH 8), and extracted with a mixture of dichloromethane/methanol. The combined organic layers were washed with water, dried (MgSO₄), concentrated in vacuo, and purified by flash column chromatography (silica gel, dichloromethane/methanol 9 / 1) to give, after crystallization from diisopropyl ether, 0.26 g (55 %) of the title product as white crystals (m.p. 265-268°C).

### 12. (7R,8R)-[6,7-Dihydroxy-2,3-dimethyl-8-phenyl-6,7,8,9-tetrahydro-3H-imidazo[4,5-h]quinoline-5-yl]-carboxylic acid dimethylamide

To a at 0°C cooled stirred solution of (7R,8R)-[7-hydroxy-2,3-dimethyl-6-oxo-8-phenyl-6,7,8,9-tetrahydro-3*H*-imidazo[4,5-h]quinoline-5-yl]-carboxylic acid dimethylamide (3.50 g, 9.24 mmol) in methanol (50 ml) was added sodium borohydride (0.70 g, 18.49 mmol). The reaction mixture was stirred for 1 h at 0°C and 5 h at room temperature, and further 18 h at 0°C. Water and a saturated ammonium chloride solution were added to neutralize (pH7-8), the aqueous phase was extracted with dichloromethane twice, the combined organic layers were washed with a small amount of water, dried (MgSO₄), and concentrated in vacuo. Purification by flash column chromatography (silica gel, dichloromethane/methanol 100 / 3) and crystallization from diisopropyl ether gave 1.97 g (56 %) of the title product as diastereomeric mixture which was not separable. MS (ESI): 381.2 (MH⁺).

### 13. (6S,7R,8R)-[6-n-Butyloxy-7-hydroxy-2,3-dimethyl-8-phenyl-6,7,8,9-tetrahydro-3H-imidazo[4,5-h]quinoline-5-yl]-carboxylic acid dimethylamide

To a at-30°C cooled stirred solution of (7R,8R)-[6,7-dihydroxy-2,3-dimethyl-8-phenyl-6,7,8,9-tetrahydro-3*H*-imidazo[4,5-h]quinoline-5-yl]-carboxylic acid dimethylamide (0.55 g, 1.44 mmol) in a mixture of n-butanol (8 ml) and dichloromethane (4 ml) was added dropwise methansulfonic acid (0.18 ml, 1.87 mmol). The reaction mixture was stirred for 30 min at -30°C, warmed to room temperature, and stirred at room temperature for 3 h. The solution was poured onto water, neutralized with a saturated sodium hydrogen carbonate solution (pH 8), and extracted with dichloromethane. The combined organic layers were washed with water, dried (MgSO₄), concentrated in vacuo, and purified by flash column chromatography (silica gel, dichloromethane/methanol 100/3) to give, after crystallization from diisopropyl ether, 0.17 g (26 %) of the title product as white crystals.
¹H-NMR (200MHz, d₆-DMSO): δ [ppm] = 0.71 (t, 3H), 0.79-1.20 (m, 4H), 2.52 (s, 3H), 2.77 (s, 3H), 2.95 (s, 3H), 3.14-3.30 (m, 1H), 3.53-3.76 (m, 4H), 3.99-4.12 (m, 1H), 4.32-4.62 (m, 1H), 5.00 (d, 1H), 5.69 (s, 1H), 6.57 (s, 1H), 7.09-7.38 (m, 5H).

### 14. (6R,7R,8R)-[6-n-Butyloxy-7-hydroxy-2,3-dimethyl-8-phenyl-6,7,8,9-tetrahydro-3H-imidazo[4,5-h]quinoline-5-yl]-carboxylic acid dimethylamide

To a at-30°C cooled stirred solution of (7R,8R)-[6,7-dihydroxy-2,3-dimethyl-8-phenyl-6,7,8,9-tetrahydro-3*H*-imidazo[4,5-h]quinoline-5-yl]-carboxylic acid dimethylamide (0.55 g, 1.44 mmol) in a mixture of n-butanol (8 ml) and dichloromethane (4 ml) was added dropwise methansulfonic acid (0.18 ml, 1.87 mmol). The reaction mixture was stirred for 30 min at -30°C, warmed to room temperature, and stirred at room temperature for 3 h. The solution was poured onto water, neutralized with a saturated sodium hydrogen carbonate solution (pH 8), and extracted with dichloromethane. The combined organic layers were washed with water, dried (MgSO₄), concentrated in vacuo, and purified by flash column chromatography (silica gel, dichloromethane/methanol 100/3) to give, after crystallization from diisopropyl ether, 0.30 g (48 %) of the title product as white crystals.
¹H-NMR (200MHz, d₆-DMSO): δ [ppm] = 0.86 (t, 3H), 1.15-1.61 (m, 4H), 2.46 (s, 3H), 2.84 (s, 3H), 3.01 (s, 3H), 3.30-3.47 (m, 1H), 3.52-3.72 (m, 4H), 3.74-3.95 (m, 1H), 4.36-4.59 (m, 2H), 4.69 (d, 1H), 5.53 (s, 1H), 6.56 (s, 1H), 7.21-7.48 (m, 5H).

### 15. (7R,8R)-[7-Hydroxy-2,3-dimethyl-8-phenyl-6,7,8,9-tetrahydro-3H-imidazo[4,5-h]quinoline-5-yl]-carboxylic acid dimethylamide

To a suspension of (7R,8R)-[6,7-dihydroxy-2,3-dimethyl-8-phenyl-6,7,8,9-tetrahydro-3H-imidazo[4,5-h]quinoline-5-yl]-carboxylic acid dimethylamide (1.80 g, 4.73 mmol) in dichloromethane (20 ml) was added trifluoroacetic acid (4.30 ml). A clear green solution resulted, triethylsilane (12.00 ml) was added, and the solution was stirred for 3 h at room temperature. The solution was poured onto water, neutralized with a sodium hydroxide solution (2N) (pH 8), and extracted with dichloromethane. The combined organic layers were washed with water, dried (MgS0₄), concentrated in vacuo, and purified by flash column chromatography (silica gel, dichloromethane/methanol 100 / 3) to give, after crystallization from diisopropyl ether, 1.30 g (75 %) of the title product as white crystals.
¹H-NMR (200MHz, d₆-DMSO): δ [ppm] = 2.33-2.55 (m, 5H), 2.74 (s, 3H), 2.97 (s, 3H), 3.65 (s, 3H), 3.95 (quintett, 1H). 3.30-3.38 (m, 1H), 4.88 (d, 1H), 5.80 (d, 1H), 6.52 (s, 1H). 7.14-7.42 (m, 5H).

### 16. (7R,8R)-[2,3-Dimethyl-7-methoxy-8-phenyl-6,7,8,9-tetrahydro-3H-imidazo[4,5-h]quinoline-5-yl]-carboxylic acid dimethylamide

To a suspension of sodium hydride (0.06 g, 1.50 mmol) in dimethylformaride (5 ml) (7R,8R)-[7-hydroxy-2,3-dimethyl-8-phenyl-6,7,8,9-tetrahydro-3*H*-imidazo[4,5-h]quinoline-5-yl]-carboxylic acid dimethylamide (0.50 g, 1.37 mmol) was added at 0°C. The resulting yellow solution was stirred for 1 h at 0°C and methyl iodide (0.09 ml, 1.50 mmol) was added. After stirring at 0°C for 1 h, a saturated solution of ammonium chloride was added, and the aqueous phase was extracted with dichloromethane twice. The combined organic layers were washed with water, dried (MgSO₄), concentrated in vacuo, and purified by flash column chromatography (silica gel, dichloromethane/methanol 100/3) to give, after crystallization from diisopropyl ether, 0.27 g (51 %) of the title product as white crystals.
¹H-NMR (200MHz, d₆-DMSO): δ [ppm] = 2.23-2.42 (m, 2H), 2.49 (s, 3H), 2.71 (s, 3H), 2.95 (s, 3H), 3.27 (s, 3H), 3.65 (s, 3H), 3.72 (q, 1H), 4.65 (t, 1H), 6.18 (d, 1H), 6.48 (s, 1H), 7.13-7.38 (m, 5H).

### II. Starting compounds and intermediates

### A. 4-Acetylamino-3-hydroxy-5-oxo-cyclohex-3-en carboxylic acid ethylester

4-(4-bromo-phenylazo)-3-hydroxy-5-oxo-cyclohex-3-en carboxylic acid ethylester (73.0 g, 198 mmol) was suspended in a mixture of acetic anhydride (113 ml) and glacial acetic acid (730 ml). Zinc powder (78 g, 1.193 mol) was added portionwise at room temperature. The reaction mixture was stirred for 4 h, diluted with dioxan, filtered over a pad of silica gel, and concentrated. The residue was taken off in dichloromethane, the precipitate (4-bromoacetanilide) was filtered off, and the filtrate was concentrated. It resulted a white solid which was purified by flash column chromatography (silica gel, toluene/dioxan 7 /3) to give, after crystallization from diisopropyl ether, 40.0 g (84 %) of the title product as white crystals. m.p. 111.5-113.5°C.

### B. 4,5,6,7-Tetrahydro-2,3-dimethyl-7-oxo-3H-benzimidazol-5-carboxylic acid ethyl ester

To a solution of 4-acetylamino-3-hydroxy-5-oxo-cyclohex-3-en carboxylic acid ethylester (30.0 g, 124 mmol) in toluene (250 ml) was added a solution of methyl amine in tetrahydrofuran (2N) (75 ml, 150 mmol) and glacial acetic acid (30 ml). The reaction mixture was transferred into an autoclave and heated for 2 h at 180°C. After cooling down the solvent is removed, and the residue is purified by flash column chromatography (silica gel, dichloromethane/methanol 9 /1) to give, after crystallization from diisopropyl ether, 25.0 g (85 %) of the title product as white crystals. m.p. 147.5-150.0°C.

### C. 4,5,6,7-Tetrahydro-2,3-dimethyl-7-oxo-3H-benzimidazol-5-carboxylic acid methylamide

4,5,6,7-tetrahydro-2,3-dimethyl-7-oxo-3*H*-benzimidazol-5-carboxylic acid ethyl ester (23.0 g, 97.4 mmol) was dissolved in a solution of methyl amine in water (40%) (160 ml), transferred into an autoclave, and heated at 150 °C for 3.5 h. After cooling down, water and remaining methyl amine were removed by distillation to form a brownish oil as raw product. Purification of this crude product was carried out by flash column chromatography (silica gel, toluene/dioxan/methanol 3 / 1 / 1). The title product was crystallized from diisopropyl ether delivering 15.5 g (73%) white crystals of the title compound (m.p. 194-196°C).

### D. (2R,3R)-3-Amino-2-(tert.-butyl-dimethyl-silanyloxy)-3-phenyl propionic acid ethyl ester

(R, R)-phenylisoserine ethyl ester (1323 g, 4.06 mole) was dissolved in dichloromethane (6.6 1). To this solution, imidazole (397.4 g) and *t*-butyldimethylsilyl chloride (724 g) were added. The mixture was stirred for 16 h at room temperature. The reaction mixture was washed subsequently with 61 and 41 of water. The resulting clear dichloromethane layer was dried over sodium sulphate, filtered and concentrated under reduced pressure. The obtained 1509 g of the title compound was used as such for the next reaction step without further purification.

### E. (7R,8R)-[7-Hydroxy-2,3-dimethyl-6-oxo-8-phenyl-4,5,6,7,8,9-hexahydro-3H-imidazo[4,5-h]quinoline-5-yl]-carboxylic acid methylamide

The suspension of 4,5,6,7-tetrahydro-2,3-dimethyl-7-oxo-3*H*-benzimidazol-5-carboxylic acid methylamide (11.0 g, 49.7 mmol) and (2R,3R)-3-amino-2-(*tert*.-butyl-dimethyl-silanyloxy)-3-phenyl propionic acid ethyl ester (16.9 g, 52.2 mmol) in 2-methoxyethanol (90 ml) was heated at 150 °C for 4 days. After cooling down, the solvent was removed by distillation, the remaining oil was dissolved in dichloromethane, and washed with a solution of sodium bicarbonate. The aqueous phase was reextracted with dichloromethane. The combined organic solutions were washed again with a small amount of water, dried (MgSO₄), and the solvent was removed. Purification of the raw product was carried out by flash column chromatography (silica gel, dichloromethane/methanol 100 / 3) delivering after crystallization from diisopropyl ether 10.33 g (64%) of the title compound as white crystals. MS (ESI): 367.1 (MH⁺)

### F. (7R,8R)-[7-Acetoxy-2,3-dimethyl-6-oxo-8-phenyl-4,5,6,7,8,9-hexahydro-3H-imidazo[4,5-h]quinoline-5-yl]-carboxylic acid methylamide

To the suspension of (7R,8R)-[7-hydroxy-2,3-dimethyl-6-oxo-8-phenyl-4,5,6,7,8,9-hexahydro-3*H-*imidazo[4,5-h]quinoline-5-yl]-carboxylic acid methylamide (4.00 g, 10.91 mmol) in acetic anhydride (20 ml) was added methane sulfonic acid (0.6 ml). A yellow solution was formed which was stirred for 1 h at room temperature followed by the addition of a second portion of methanesulfonic acid (0.3 ml). The reaction mixture was stirred for further 2 h at room temperature. Acetic anhydride was removed by distillation, the remaining oil was dissolved in dichloromethane, and the solution was neutralized by addition of an aqueous solution of sodium bicarbonate (pH 8). Drying of the organic solution (MgSO₄), removal of the solvent, and purification by flash column chromatography (silica gel, dichloromethane/methanol 100/3) delivered an oil which was crystallized from diisopropyl ether. 3.23 g (72 %) of the title product were isolated as white crystals. MS (ESI): 410.1 (MH⁺)

### G. (7R,8R)-[2,3-Dimethyl-6-oxo-8-phenyl-7-pivaloyloxy-4,5,6,7,8,9-hexahydro-3H-imidazo[4,5-h]quinoline-5-yl]-carboxylic acid methylamide

To the suspension of (7R,8R)-[7-hydroxy-2,3-dimethyl-6-oxo-8-phenyl-4,5,6,7,8,9-hexahydro-3*H-*imidazo[4,5-h]quinoline-5-yl]-carboxylic acid methylamide (0.30 g, 0.82 mmol) in dichloromethane (10 ml) were added pivalic anhydride (0.33 ml, 1.64 mmol) and methanesulfonic acid (0.1 ml). After reflux for 5 h further pivalic anhydride (0.10 ml, 0.55 mmol) and methanesulfonic acid (0.1 ml) were added and reflux was continued for further 6 h. The reaction mixture was neutralized by addition of an aqueous solution of sodium bicarbonate (pH 8), extracted with dichloromethane, the combined organic phases were washed with water, and dried (MgSO₄). After removal of the solvent, purification by flash column chromatography (silica gel, dichloromethane/methanol 20/1) and crystallization from diisopropyl ether, 0.168 g (46%) of the title compound were isolated as white crystals. MS (ESI): 451.1 (MH⁺).

### H. (7R,8R)-[7-Acetoxy-2,3-dimethyl-6-oxo-8-phenyt-6,7,8,9-tetrahydro-3H-imidazo[4,5-h]quinoline-5-yl]-carboxylic acid methylamide

(7R,8R)-[7-Acetoxy-2,3-dimethyl-6-oxo-8-phenyl-4,5,6,7,8,9-hexahydro-3H-imidazo[4,5-h]quinoline-5-yl]-carboxylic acid methylamide (3.00 g, 7.34 mmol) was suspended in ethyl acetate (80 ml), and 2,3-dichloro-4,5-dicyano benzoquinone (3.33 g, 14.69 mmol) were added in portions at room temperature. A dark solution resulted which was stirred for 24 h at room temperature. The reaction mixture was poured onto an aqueous solution of sodium bicarbonate, the phases were separated, and the aqueous phase was extracted with dichloromethane. The combined organic phases were washed with water, dried (MgS0₄), the solvent was removed, and the raw product was purified by flash column chromatography (silica gel, toluene/dioxan/methanol 6 / 3.5 / 0.5). The title product was crystallized from diisopropyl ether yielding 1.30 g (44 %) of pale yellow crystals.
¹H-NMR (200MHz, d₆-DMSO): δ [ppm] = 1.96 (s, 3H), 2.49 (s, 3H), 2.69 (d, 3H), 3.71 (s, 3H), 4.83 (d, 1H), 5,47 (d, 1H), 6.70 (s, 1H), 7.07 (s, 1H), 7.35-7.48 (m, 5H), 7.75 (d, 1H).

### 1. (7R,8R)-[7-Hydroxy-2,3-dimethyl-6-oxo-8-phenyl-6,7,8,9-tetrahydro-3H-imidazo[4,5-h]quinoline-5-yl]-carboxylic acid methylamide

Hydrazine hydrate (0.62 g, 12.3 mmol) was added at room temperature to the suspension of (7R,8R)-[7-acetoxy-2,3-dimethyl-6-oxo-8-phenyl-6,7,8,9-tetrahydro-*3H*-imidazo[4,5-h]quinoline-5-yl]-carboxylic acid methylamide (2.50 g, 6.15 mmol) in methanol (25 ml). The reaction mixture was stirred for 5 h at room temperature, water (15 ml) was added, and stirring was continued for further 60 min. White crystals precipitated which were filtered off, washed with water and dried at 40°C in vacuo over potassium hydroxide for 18 h. The resulting title compound, 1.94 g (87%), did not afford further purifications.
¹H-NMR (200MHz, CDCl₃): δ [ppm] = 2.56 (s, 3H), 3.07 (d, 3H), 3.69 (s, 3H), 4.59 (dd, 2H), 5.86 (d, 1H), 6.77 (s, 1H), 7.26 (s, 1H), 7.39-7.48 (m, 3H), 7.58-7.62 (m, 2H).

### J. 3-Hydroxy-5-oxo-4-phenylazo-cyclohex-3-enecarboxylic acid dimethylamide

The suspension of 3-hydroxy-5-oxo-4-phenylazo-cyclohex-3-enecarboxylic acid (30.0 g, 115 mmol) and *O*-(1H-benzotriazol-1-yl)-*N*,*N*,*N'*,*N'*-tetramethyluronium tetrafluoroborate (TBTU) (38.9 g, 121 mmol) in dichloromethane (400 ml) was heated to reflux for 2 h. After cooling to room temperature, the solution of dimethylamine in tetrahydrofuran (2N) (575 ml, 1.15 mol) was added and the reaction mixture was stirred for 2 h at room temperature. Hydrochloric acid (2N) (500 ml) was added to acidify the reaction mixture (pH 1-2), and the aqueous phase was extracted with dichloromethane. The combined organic phases were washed with an aqueous solution of sodium bicarbonate, dried (MgS0₄), the solvent was removed, and the raw product was recrystallized from diisopropyl ether. 13.45 g (41 %) of the title compound were isolated as pale yellow crystals (m.p. 179-181 °C).

### K. 4-Acetylamino-3-hydroxy-5-oxo-cyclohex-3-enecarboxylic acid dimethylamide

3-Hydroxy-5-oxo-4-phenylazo-cydohex-3-en carboxylic acid dimethylamid (34.0 g, 118 mmol) was suspended in a mixture of acetic anhydride (68 ml, 710 mmol) and glacial acetic acid (340 ml). Zinc powder (46.4 g, 710 mmol) was added por6onwise keeping the temperature under 60°C. The reaction mixture was stirred for 1 h, diluted with dioxan, filtered over a pad of silica gel, and concentrated. The residue was purified by flash column chromatography (silica gel, toluene/dioxan/methanol 6 / 3 /1) to give, after crystallization from diisopropyl ether, 22.3 g (79 %) of the title product as white crystals (m.p. 162-164°C).

### L. 4,5,6,7-Tetrahydro-2,3-dimethyl-7-oxo-3H-benzimidazol-5-carboxylic acid dimethylamide

To a solution of 4-acetylamino-3-hydroxy-5-oxo-cyclohex-3-encarboxylic acid dimethylamide (3.5 g, 14.56 mmol) in toluene (25 ml) were added a solution of methylamine in tetrahydrofuran (2N) (15 ml, 30 mmol) and glacial acetic acid (3.5 ml). The reaction mixture was transferred into an autoclave and heated for 2 h at 180°C. After cooling down the solvent is removed, and the residue is purified by flash column chromatography (silica gel, dichloromethane/methanol 100/1, then 10/1) to give, after crystallization from diethyl ether, 2.5 g (73 %) of the title product as white crystals (m.p. 190-194°C).

### M. (7R,8R)-[7-Hydroxy-2,3-dimethyl-6-oxo-8-phenyl-4,5,6,7,8,9-hexahydro-3H-imidazo[4,5-h]quinoline-5-yl]-carboxylic acid dimethylamide

The suspension of 4,5,6,7-tetrahydro-2,3-dimethyl-7-oxo-3*H*-benzimidazol-5-carboxylic acid dimethylamide (8.5 g, 36.12 mmol) and (2R,3R)-3-amino-2-(*tert*.-butyl-dimethyl-silanyloxy)-3-phenyl propionic acid ethyl ester (12.3 g, 37.93 mmol) in 2-methoxyethanol (85 ml) was heated at 150 °C for 5 days, further (2R,3R)-3-amino-2-(*tert*.-butyl-dimethyl-silanyloxy)-3-phenyl propionic acid ethyl ester (3.00 g, 9.25 mmol) was added, and the reaction mixture was heated at 150 °C for further 2 days. After cooling down, the solvent was removed by distillation, the remaining oil was dissolved in dichloromethane and water, and the aqueous phase was extracted with dichloromethane. Washing of of the combined organic phases with water, drying (MgSO₄), removal of the solvent, and purification of the raw product by flash column chromatography (silica gel, dichloromethane/methanol 100 / 3, then 20 :1) delivered after crystallization from diisopropyl ether 6.00 g (44 %) of the title compound as white crystals (m.p. 224-226°C).

### N. (7R,8R)-[7-Acetoxy-2,3-dimethyl-6-oxo-8-phenyl-4,5,6,7,8,9-hexahydro-3H-imidazo[4,5-h]quinoline-5-yl]-carboxylic acid dimethylamide

To the suspension of (7R,8R)-[7-hydroxy-2,3-dimethyl-6-oxo-8-phenyl-4,5,6,7,8,9-hexahydro-3*H-*imidazo[4,5-h]quinoline-5-yl]-carboxylic acid dimethylamide (9.00 g, 23.65 mmol) in acetic anhydride (90 ml) was added methane sulfonic acid (3.5 ml). A pale yellow solution was formed which was stirred for 2 h at room temperature. Acetic anhydride was removed by distillation, the remaining oil was dissolved in dichloromethane, and the solution was neutralized by addition of an aqueous solution of sodium bicarbonate (pH 8). Drying of the organic solution (MgSO₄), removal of the solvent, and purification by flash column chromatography (silica gel, dichloromethane/methanol 100 / 3) delivered a pale yellow oil which was crystallized from diisopropyl ether. 7.54 g (75 %) of the title product were isolated as pale yellow crystals (m.p. 198-207°C).

### O. (7R,8R)-[7-Acetoxy-2,3-dimethyl-6-oxo-8-phenyl-6,7,8,9-tetrahydro-3H-imidazo[4,5-h]quinoline-5-yl]-carboxylic acid dimethylamide

(7R,8R)-[7-Acetoxy-2,3-dimethyl-6-oxo-8-phenyl-4.,5,6,7,8,9-hexahydro-3H imidazo[4,5-h]quinoline-5-yl]-carboxylic acid dimethylamide (7.40 g, 17.51 mmol) were suspended in ethyl acetate (120 ml) and 2,3-dichloro-4,5-dicyano benzoquinone (7.95 g, 35.00 mmol) were added in portions at room temperature. A dark solution resulted which was stirred for 24 h at room temperature. The reaction mixture was poured onto an aqueous solution of sodium bicarbonate, the phases were separated, and the aqueous phase was extracted with dichloromethane. The combined organic phases were washed with water, dried (MgSO₄), the solvent was removed, and the raw product was purified by flash column chromatography (silica gel, toluene/dioxan/methanol 6/3.5 /0.5). The title product was crystallized from diisopropyl ether yielding 5.03 g (68 %) of yellow crystals (m.p. 219-222°C).

### P. (7R,8R)-[7-Hydroxy-2,3-dimethyl-6-oxo-8-phenyl-6,7,8,9 tetrahydro-3H-imidazo(4,5-h]quinoline-5-yl]-carboxylic acid dimethylamide

Hydrazine hydrate (1.17 g, 23.3 mmol) were added at room temperature to the suspension of (7R,8R)-[7-acetoxy-2,3-dimethyl-6-oxo-8-phenyl-6,7,8,9-tetrahydro-3*H*-imidazo[4,5-h]quinoline-5-yl]-carboxylic acid dimethylamide (4.90 g, 11.65 mmol) in methanol (50 ml). The reaction mixture was stirred for 18 h at room temperature, poured onto water, and extracted with dichloromethane. The combined organic phases were washed with water, dried (MgSO₄), and the solvent was removed. A yellow oil resulted which was crystallized from diisopropyl ether yielding 4:29 g (97 %) of yellow crystals.
¹H-NMR (200MHz, d₆-DMSO): δ [ppm] = 2.50 (s, 3H), 2.60 (d, 3H), 2.94 (d, 3H), 3.69 (s, 3H), 4.11-4.28 (m, 1H), 4.59 (d, 2H), 5.51 (dd, 1H), 6.65 (d, 1H), 6.96 (d, 1H), 7.23-7.47 (m, 5H).

### Commercial Utility

The compounds of the formulae 1, 1 a and 1 b and their pharmacologically acceptable salts (= active compounds according to the invention) have valuable pharmacological properties which make them commercially utilizable. In particular, they exhibit marked inhibition of gastric acid secretion and an excellent gastric and intestinal protective action in warm-blooded animals, in particular humans. In this connection, the active compounds according to the invention are distinguished by a high selectivity of action, an advantageous duration of action, a particularly good enteral activity, the absence of significant side effects and a large therapeutic range.

"Gastric and intestinal protection" in this connection is understood as meaning the prevention and treatment of gastrointestinal diseases, in particular of gastrointestinal inflammatory diseases and lesions (such as, for example, gastric ulcer, peptic ulcer, including peptic ulcer bleeding, duodenal ulcer, gastritis, hyperacidic or medicament-related functional dyspepsia), which can be caused, for example, by microorganisms (e.g. Helicobacter pylori), bacterial toxins, medicaments (e.g. certain antiinflammatories and antirheumatics, such as NSAIDs and COX-inhibitors), chemicals (e.g. ethanol), gastric acid or stress situations. "Gastric and intestinal protection" is understood to include, according to general knowledge, gastroesophageal reflux disease (GERD), the symptoms of which include, but are not limited to, heartburn and/or acid regurgitation.

In their excellent properties, the active compounds according to the invention surprisingly prove to be clearly superior to the compounds known from the prior art in various models in which the antiulcerogenic and the antisecretory properties are determined. On account of these properties, the active compounds according to the invention are outstandingly suitable for use in human and veterinary medicine, where they are used, in particular, for the treatment and/or prophylaxis of disorders of the stomach and/or intestine.

A further subject of the invention are therefore the active compounds according to the invention for use in the treatment and/or prophylaxis of the abovementioned diseases.

The invention likewise includes the use of the active compounds according to the invention for the production of medicaments which are employed for the treatment and/or prophylaxis of the abovementioned diseases.

The invention furthermore includes the use of the active compounds according to the invention for the treatment and/or prophylaxis of the abovementioned diseases.

A further subject of the invention are medicaments which comprise one or more active compounds according to the invention.

The medicaments are prepared by processes which are known per se and familiar to the person skilled in the art. As medicaments, the active compounds according to the invention (= active compounds) are either employed as such, or preferably in combination with suitable pharmaceutical auxiliaries or excipients in the form of tablets, coated tablets, capsules, suppositories, patches (e.g. as TTS), emulsions, suspensions or solutions, the active compound content advantageously being between 0.1 and 95% and it being possible to obtain a pharmaceutical administration form exactly adapted to the active compound and/or to the desired onset and/or duration of action (e.g. a sustained-release form or an enteric form) by means of the appropriate selection of the auxiliaries and excipients.

The auxiliaries and excipients which are suitable for the desired pharmaceutical formulations are known to the person skilled in the art on the basis of his/her expert knowledge. In addition to solvents, gel-forming agents, suppository bases, tablet auxiliaries and other active compound excipients, it is possible to use, for example, antioxidants, dispersants, emulsifiers, antifoams, flavor corrigents, preservatives, solubilizers, colorants or, in particular, permeation promoters and complexing agents (e.g. cyclodextrins).

The active compounds can be administered orally, parenterally or percutaneously.

In general, it has proven advantageous in human medicine to administer the active compound(s) in the case of oral administration in a daily dose of approximately 0.01 to approximately 20, preferably 0.05 to 5, in particular 0.1 to 1.5, mg/kg of body weight, if appropriate in the form of several, preferably 1 to 4, individual doses to achieve the desired result. In the case of a parenteral treatment, similar or (in particular in the case of the intravenous administration of the active compounds), as a rule, lower doses can be used. The establishment of the optimal dose and manner of administration of the active compounds necessary in each case can easily be carried out by any person skilled in the art on the basis of his/her expert knowledge.

If the active compounds according to the invention and/or their salts are to be used for the treatment of the abovementioned diseases, the pharmaceutical preparations can also contain one or more pharmacologically active constituents of other groups of medicaments, for example: tranquillizers (for example from the group of the benzodiazepines, for example diazepam), spasmolytics (for example, bietamiverine or camylofine), anticholinergics (for example, oxyphencyclimine or phencarbamide), local anesthetics, (for example, tetracaine or procaine), and, if appropriate, also enzymes, vitamins or amino acids.

To be emphasized in this connection is in particular the combination of the active compounds according to the invention with pharmaceuticals which inhibit acid secretion, such as, for example, H₂ blockers (e.g. cimetidine, ranitidine), H⁺/K⁺ ATPase inhibitors (e.g. omeprazole, pantoprazole), or further with so-called peripheral anticholinergics (e.g. pirenzepine, telenzepine) and with gastrin antagonists with the aim of increasing the principal action in an additive or super-additive sense and/or of eliminating or of decreasing the side effects, or further the combination with antibacterially active substances (such as, for example, cephalosporins, tetracyclines, penicillins, macrolides, nitroimidazoles or alternatively bismuth salts) for the control of Helicobacter pylori. Suitable antibacterial co-components which may be mentioned are, for example, mezlocillin, ampicillin, amoxicillin, cefalothin, cefoxitin, cefotaxime, imipenem, gentamycin, amikacin, erythromycin, ciprofloxacin, metronidazole, clarithromycin, azithromycin and combinations thereof (for example clarithromycin + metronidazole).

In view of their excellent gastric and intestinal protection action, the active compounds according to the invention are suited for a free or fixed combination with those medicaments (e.g. certain antiinflammatories and antirheumatics, such as NSAIDs), which are known to have a certain ulcerogenic potency. In addition, the compounds of formula 1 are suited for a free or fixed combination with motility-modifying drugs.

### Pharmacology

The excellent gastric protective action and the gastric acid secretion-inhibiting action of the compounds according to the invention can be demonstrated in investigations on animal experimental models. The compounds according to the invention investigated in the model mentioned below have been provided with numbers which correspond to the numbers of these compounds in the examples.

### Testing of the secretion-inhibiting action on the perfused rat stomach

In Table A which follows, the influence of the compounds of the formula 1 according to the invention on the pentagastrin-stimulated acid secretion of the perfused rat stomach after intraduodenal administration in vivo is shown.

**Table A**

| No. | Dose (µmol/kg) i.d. | inhibition of acid secretion (%) |
|---|---|---|
| **1** | 3 | > 15 |
| **2** | 3 | >15 |
| **3** | 3 | >15 |
| **4** | 3 | > 15 |
| **7** | 3 | > 15 |
| **10** | 3 | > 15 |
| **11** | 3 | > 15 |
| **12** | 3 | > 15 |
| **13** | 3 | > 15 |
| **14** | 3 | > 15 |
| **15** | 3 | > 15 |

### Methodology

The abdomen of anesthetized rats (CD rat, female, 200-250 g; 1.5 g/kg i.m. urethane) was opened after tracheotomy by a median upper abdominal incision and a PVC catheter was fixed transorally in the esophagus and another via the pylorus such that the ends of the tubes just projected into the gastric lumen. The catheter leading from the pylorus led outward into the right abdominal wall through a side opening.

After thorough rinsing (about 50-100 ml), warm (37°C) physiological NaCI solution was continuously passed through the stomach (0.5 mi/min, pH 6.8-6.9; Braun-Unita I). The pH (pH meter 632, glass electrode EA 147; φ = 5 mm, Metrohm) and, by titration with a freshly prepared 0.01 N NaOH solution to pH 7 (Dosimat 665 Metrohm), the secreted HCl were determined in the effluent in each case collected at an interval of 15 minutes.

The gastric secretion was stimulated by continuous infusion of 1 µg/kg (= 1.65 ml/h) of i.v. pentagastrin (left femoral vein) about 30 min after the end of the operation (i.e. after determination of 2 preliminary fractions). The substances to be tested were administered intraduodenally in a 2.5 ml/kg liquid volume 60 min after the start of the continuous pentagastrin infusion. The body temperature of the animals was kept at a constant 37.8-38°C by infrared irradiation and heat pads (automatic, stepless control by means of a rectal temperature sensor).

## Claims

1. A compound of the formula 1, 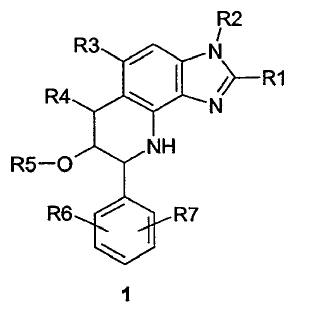 in which
R1 is hydrogen, halogen, 1-4C-alkyl, 3-7C-cycloalkyl, 3-7C-cycloalkyl-1-4C-alkyl, 1-4C-alkoxy, 1-4C-alkoxy-1-4C-alkyl, 1-4C-alkoxycarbonyl, 2-4C-alkenyl, 2-4C-alkynyl, fluoro-1-4C-alkyl, fluoro-1-4C-alkoxy-1-4C-alkyl or hydroxy-1-4C-alkyl,
R2 is hydrogen, 1-4C-alkyl, 3-7C-cycloalkyl, 3-7C-cycloalkyl-14C-alkyl, 1-4C-alkoxy-1-4C-alkyl,2-4C-alkenyl, 2-4C-alkynyl, fluoro-1-4C-alkyl or hydroxy-1-4C-alkyl,
R3 is halogen, fluoro-1-4C-alkyl, carboxyl, -CO-1-4Galkoxy, hydroxy-1-4C-alkyl, 1-4C-alkoxy-1-4C-alkyl, 1-4C-alkoxy-1-4C-alkoxy-1-4C-alkyl, fluoro-1-4C-alkoxy-1-4C-alkyl, cyano, the group -CO-NR31 R32, the group SO₂-NR31R32or the group Het,
where
R31 is hydrogen, hydroxyl, 1-7C-alkyl, hydroxy-1-4C-alkyl, 1-4C-alkoxy-1-4C-alkyl, 3-7C-cycloalkyl, or amino and
R32 is hydrogen, 1-7C-alkyl, hydroxy-1-4C-alkyl or 1-4C-alkoxy-1-4C-alkyl,
or where
R31 and R32 together, including the nitrogen atom to which both are bonded, are a pyrrolidino, piperidino, piperazino, N-1-4C-alkylpiperazino, morpholino, aziridino or azetidino group and Het is a heterocyclic residue, substituted by R33, R34 and R35, selected from the group consisting of oxadiazol, dihydrooxazol, dihydroimidazol, oxazol, imidazol, isoxazol, dihydroisoxazol, pyrazol and tetrazol,
where
R33 is hydrogen, 1-4C-alkyl, hydroxy-1-4C-alkyl, 1-4C-alkoxy, 2-4C-alkenyloxy, 1-4C-alkylcarbonyl, carboxy, 1-4C-alkoxycarbonyl, carboxy-1-4C-alkyl, 1-4C-alkoxycarbonyl-1-4C-alkyl, halogen, hydroxy, aryl, aryl-1-4C-alkyl, aryl-oxy, aryl-1-4C-alkoxy, trifluoromethyl, nitro, amino, mono- or di-1-4C-alkylamino, 1-4C-alkylcarbonylamino, 1-4Calkoxycarbonylamino, 1-4C-alkoxy-1-4C-alkoxycarbonylamino or sulfonyl,
R34 is hydrogen, 1-4C-alkyl, 1-4C-alkoxy, 1-4C-alkoxycarbonyl, halogen, trifluoromethyl or hydroxy,
R35 is hydrogen, 1-4C-alkyl, 1-4C-alkoxy, 1-4.C-alkoxycarbonyl, halogen, trifluoromethyl or hydroxy,
R4 is hydrogen, halogen, 1-4C-alkyl, 3-7C-cycloalkyl, 3-7C-cycloalkyl-1-4C-alkyl, 1-4C-alkoxy-1-4C-alkyl, fluoro-1-4C-alkyl, fluoro-1-4C-alkoxy-1-4C-alkyl, hydroxy-1-4C-alkyl, hydroxy, 1-4C-alkoxy, 3-7C-cycloalkoxy, 3-7C-cycloalkyl-1-4C-alkoxy, 1-4C-alkoxy-1-4C-alkoxy, fluoro-1-4C-alkoxy, fluoro-1-4C-alkoxy-1-4C-alkoxy, hydroxy-1-4C-alkoxy, 2-4C-alkenyloxy or 2-4C-alkynyloxy
R5 is hydrogen, 1-4C-alkyl, 3-7C-cycloalkyl, 3-7C-cycloalkyl-1-4C-alkyl, 1-4C-alkoxy-1-4C-alkyl, fluoro-1-4C-alkyl, fluoro-1-4C-alkoxy- 1-4C-alkyl or hydroxy-1-4C-alkyl,
R6 is hydrogen, halogen, 1-4C-alkyl or fluoro-1-4C-alkyl,
R7 is hydrogen, halogen, 1-4C-alkyl or fluoro-1-4C-alkyl,
and wherein
aryl is phenyl or substituted phenyl with one, two or three identical or different substituents from the group of 1-4C-alkyl, 1-4C-alkoxy, carboxy, 1-4C-alkoxycarbonyl, halogen, trifluoromethyl, nitro, trifluoromethoxy, hydroxy and cyano,
and its salts.

2. A compound of the formula 1 as claimed in claim 1, in which
R1 is hydrogen, 1-4C-alkyl or 3-7C-cycloalkyl,
R2 is hydrogen or 1-4C-alkyl,
R3 is halogen, fluoro-1-4C-alkyl, carboxyl, -CO-1-4C-alkoxy, hydroxy-1-4C-alkyl, 1-4C-alkoxy-1-4C-alkyl, 1-4C-alkoxy-1-4C-alkoxy-1-4C-alkyl, fluoro-1-4C-alkoxy-1-4C-alkyl or the group -CO-NR31R32,
where
R31 is hydrogen, hydroxyl, 1-7C-alkyl, hydroxy-1-4C-alkyl, 1-4C-alkoxy-1-4C-alkyl or 3-7C-cycloalkyl and
R32 is hydrogen, 1-7C-alkyl, hydroxy-1-4C-alkyl or 1-4C-alkoxy-1-4C-alkyl,
or where
R31 and R32 together, including the nitrogen atom to which both are bonded, are a pyrrolidino, piperidino, piperazino, N-1-4C-alkylpiperazino, morpholino, aziridino or azetidino group,
R4 is hydrogen, halogen, hydroxyl, 1-4C-alkyl, 3-5C-cycloalkyl, 1-4C-alkoxy, 3-5C-cycloalkoxy, 1-4C-alkoxy-1-4C-alkoxy, fluoro-1-4C-alkoxy, fluoro-1-4C-alkoxy-1-4C-alkoxy, hydroxy-1-4C-alkoxy, 2-4C-alkenyloxy or 2-4C-alkynyloxy, and
R5 is hydrogen, 1-4C-alkyl or hydroxy-1-4C-alkyl,
R6 is hydrogen, fluoro, 1-4C-alkyl or fluoro-1-4C-alkyl,
R7 is hydrogen, fluoro, 1-4C-alkyl or fluoro-1-4C-alkyl
and its salts.

3. A compound of the formula 1 as claimed in claim 1, in which
R1 is hydrogen or 1-4C-alkyl,
R2 is 1-4C-alkyl,
R3 is carboxyl, -CO-1 -4C-alkoxy, hydroxy-1-4C-alkyl, 1-4C-alkoxy-1-4C-alkyl or the group -CO-NR31 R32,
where
R31 is hydrogen, 1-7C-alkyl, hydroxy-1-4C-alkyl, or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
R4 is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy
R5 is hydrogen,
R6 is hydrogen,
R7 is hydrogen
and its salts.

4. A compound of the formula 1 as claimed in claim 1, in which
R1 is hydrogen or 1-4C-alkyl,
R2 is 1-4C-alkyl,
R3 is carboxyl, -CO-1-4C-alkoxy, hydroxy-1-4C-alkyl, 1-4C-alkoxy-1-4C-alkyl or the group -CO-NR31 R32,
where
R31 is hydrogen, 1-7C-alkyl, hydroxy-1-4C-alkyl, or 1-4C-alkoxy-1-4C-alkyl and
R32 is hydrogen or 1-7C-alkyl
or where
R31 and R32 together, including the nitrogen atom to which both are bonded, are a pyrrolidino, morpholino, aziridino or azetidino group,
R4 is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy
R5 is hydrogen or 1-4C-alkyl,
R6 is hydrogen,
R7 is hydrogen
and its salts.

5. A compound of the formula 1 as claimed in claim 1, in which
R1 is hydrogen or 1-4C-alkyl,
R2 is 1-4C-alkyl,
R3 is carboxyl, -CO-1-4C-alkoxy or the group -CO-NR31 R32,
where
R31 is hydrogen, 1-4C-alkyl, hydroxy-1-4C-alkyl or 1-4C-alkoxy-1-4C-alkyl and
R32 is hydrogen or 1-4C-alkyl,
or where
R31 and R32 together, including the nitrogen atom to which both are bonded, are a pyrrolidino, morpholino, aziridino or azetidino group,
R4 is hydrogen, halogen, hydroxyl, 1-4C-alkyl, 3-5C-cycloalkyl, 1-4C-alkoxy, fluoro-1-4C-alkoxy, 1-4C-alkoxy-1-4C-alkoxy, 2-4C-alkenyloxy or 2-4C-alkynyloxy
R5 is hydrogen or 1-4C-alkyl,
R6 is hydrogen,
R7 is hydrogen
and its salts.

6. A compound of the formula 1 as claimed in claim 1, in which
R1 is 1-4C-alkyl,
R2 is 1-4C-alkyl,
R3 is -CO-NR31R32,
where
R31 is hydrogen or 1-4C-alkyl
R32 is hydrogen or 1-4C-alkyl,
R4 is hydrogen, hydroxyl, 1-4C-alkoxy, fluoro-1-4C-alkoxy, 1-4C-alkoxy-1-4C-alkoxy or 2-4C-alkynyloxy,
R5 is hydrogen,
R6 is hydrogen,
R7 is hydrogen
and its salts.

7. A compound of the formula 1 as claimed in claim 1, in which
in which
R1 is 1-4C-alkyl,
R2 is 1-4C-alkyl,
R3 is -CO-NR31 R32,
where
R31 is hydrogen or 1-4C-alkyl
R32 is hydrogen or 1-4C-alkyl,
R4 is hydrogen, hydroxyl, 1-4C-alkoxy, fluoro-1-4C-alkoxy, 1-4C-alkoxy-1-4C-alkoxy or 2-4C-alkynyloxy,
R5 is hydrogen or 1-4C-alkyl,
R6 is hydrogen,
R7 is hydrogen
and its salts.

8. A compound of the formula 1 as claimed in claim 1, **characterized by** the formula 1a, 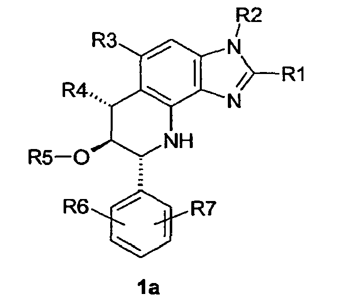 in which
R1 is hydrogen, halogen, 1-4C-alkyl, 3-7C-cycloalkyl, 3-7C-cycloalkyl-1-4C-alkyl, 1-4C-alkoxy, 1-4C-alkoxy-1-4C-alkyl, 1-4C-alkoxycarbonyl, 2-4C-alkenyl, 2-4C-alkynyl, fluoro-1-4C-alkyl, fluoro-1-4C-alkoxy-1-4C-alkyl or hydroxy-1-4C-alkyl,
R2 is hydrogen, 1-4C-alkyl, 3-7C-cycloalkyl, 3-7cycloalkyl-1-4C-alkyl, 1-4C-alkoxy-1-4C-alkyl, 2-4C-alkenyl, 2-4C-alkynyl, fluoro-1-4C-alkyl or hydroxy-1 -4C-alkyl,
R3 is halogen, fluoro-1-4C-alkyl, carboxyl, -CO-1-4C-alkoxy, hydroxy-1-4C-alkyl, 1-4C-alkoxy-1-4C-alkyl, 1-4C-alkoxy-1-4C-alkoxy-1-4C-alkyl, fluoro-1-4C-alkoxy-1-4C-alkyl, cyano, the group -CO-NR31 R32, the group SO₂-NR31 R32 or the group Het,
where
R31 is hydrogen, hydroxyl, 1-7C-alkyl, hydroxy-1-4C-alkyl, 1-4C-alkoxy-1-4C-alkyl 3-7C-cycloalkyl or amino and
R32 is hydrogen, 1-7C-alkyl, hydroxy-1-4C-alkyl or 1-4C-alkoxy-1-4C-alkyl,
or where
R31 and R32 together, including the nitrogen atom to which both are bonded, are a pyrrolidino, piperidino, piperazino, N-1-4C-alkylpiperazino, morpholino, aziridino or azetidino group and
Het is a heterocyclic residue, substituted by R33, R34 and R35, selected from the group consisting of oxadiazol, dihydrooxazol, dihydroimidazol, oxazol, imidazol, isoxazol, dihydroisoxazol, pyrazol and tetrazol,
where
R33 is hydrogen, 1-4C-alkyl, hydroxy-1-4C-alkyl, 1-4C-alkoxy, 2-4C-alkenyloxy, 1-4C-alkylcarbonyl, carboxy, 1-4C-alkoxycarbonyl, carboxy-1-4C-alkyl, 1-4C-alkoxycarbonyl-1-4C-alkyl, halogen, hydroxy, aryl, aryl-1-4C-alkyl, aryl-oxy, aryl-1-4C-alkoxy, trifluoromethyl, nitro, amino, mono- or di-1-4C-alkylamino, 1-4C-alkylcarbonylamino, 1-4Calkoxycarbonylamino, 1-4C-alkoxy-1-4C-alkoxycarbonylamino or sulfonyl,
R34 is hydrogen, 1-4C-alkyl, 1-4C-alkoxy, 1-4C-alkoxycarbonyl, halogen, trifluoromethyl or hydroxy,
R35 is hydrogen, 1-4C-alkyl, 1-4C-alkoxy, 1-4C-alkoxycarbonyl, halogen, trifluoromethyl or hydroxy,
R4 is hydrogen, halogen, 1-4C-alkyl, 3-7C-cycloalkyl, 3-7C-cycloalkyl-1-4C-alkyl, 1-4C-alkoxy-1-4C alkyl, fluoro-1-4C-alkyl, fluoro-1-4C-alkoxy-1-4C-alkyl, hydroxy-1-4C-alkyl, hydroxy, 1-4C-alkoxy, 3-7C-cycloalkoxy, 3-7C-cycloalkyl-1-4C-alkoxy, 1-4C-alkoxy-1-4C-alkoxy, fluoro-1-4C-alkoxy, fluoro-1-4C-alkoxy-1-4C-alkoxy, hydroxy-1-4C-alkoxy, 2-4C-alkenyloxy or 2-4C-alkynyloxy
R5 is hydrogen, 1-4C-alkyl, 3-7C-cycloalkyl, 3-7C-cycloalkyl-1-4C-alkyl, 1-4Galkoxy-1-4C-alkyl, fluoro-1-4C-alkyl, fluoro-1-4C-alkoxy- 1-4C-alkyl or hydroxy-1-4C-alkyl,
R6 is hydrogen, halogen, 1-4C-alkyl or fluoro-1-4C-alkyl,
R7 is hydrogen, halogen, 1-4C-alkyl or fluoro-1-4C-alkyl,
and wherein
aryl is phenyl or substituted phenyl with one, two or three identical or different substituents from the group of 1-4C-alkyl, 1-4C-alkoxy, carboxy, 1-4C-alkoxycarbonyl, halogen, trifluoromethyl, nitro, trifluoromethoxy, hydroxy and cyano,
and its salts.

9. A compound of the formula 1 b, 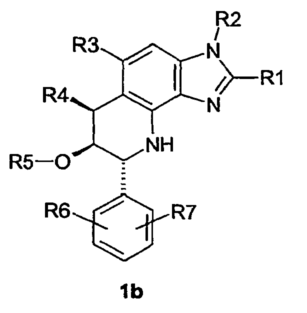 in which
R1 is hydrogen, halogen, 1-4C-alkyl, 3-7C-cycloalkyl, 3-7C-cycloalkyl-1-4C-alkyl, 1-4C-alkoxy, 1-4C-alkoxy-1-4C-alkyl, 1-4C-alkoxycarbonyl, 2-4C-alkenyl, 2-4C-alkynyl, fluoro-1-4C-alkyl, fluoro-1-4C-alkoxy-1-4C-alkyl or hydroxy-1-4C-alkyl,
R2 is hydrogen, 1-4C-alkyl, 3-7C-cycloalkyl, 3-7C-cycloalkyl-1-4C-alkyl, 1-4C-alkoxy-1-4C-alkyl, 2-4C-alkenyl, 2-4C-alkynyl, fluoro-1-4C-alkyl or hydroxy-1-4C-alkyl,
R3 is halogen, fluoro-1-4C-alkyl, carboxyl, -CO-1-4C-alkoxy, hydroxy-1-4C-alkyl, 1-4C-alkoxy-1-4C-alkyl, 1-4C-alkoxy-1-4C-alkoxy-1-4C-alkyl, fluoro-1-4C-alkoxy-1-4C-alkyl, cyano, the group -CO-NR31 R32, the group SO₂-NR31 R32 or the group Het,
where
R31 is hydrogen, hydroxyl, 1-7C-alkyl, hydroxy-1-4C-alkyl, 1-4C-alkoxy-1-4C-alkyl, 3-7C-cycloalkyl or amino and
R32 is hydrogen, 1-7C-alkyl, hydroxy-1-4C-alkyl or 1-4C-alkoxy-1-4C-alkyl,
or where
R31 and R32 together, including the nitrogen atom to which both are bonded, are a pyrrolidino, piperidino, piperazino, N-1-4C-alkylpiperazino, morpholino, aziridino or azetidino group and Het is a heterocyclic residue, substituted by R33, R34 and R35, selected from the group consisting of oxadiazol, dihydrooxazol, dihydroimidazol, oxazol, imidazol, isoxazol, dihydroisoxazol, pyrazol and tetrazol,
where
R33 is hydrogen, 1-4C-alkyl, hydroxy-1-4C-alkyl, 1-4C-alkoxy, 2-4C-alkenyloxy, 1-4C-alkylcarbonyl, carboxy, 1-4C-alkoxycarbonyl, carboxy-1-4C-alkyl, 1-4C-alkoxycarbonyl-1-4C-alkyl, halogen, hydroxy, aryl, aryl-1-4C-alkyl, aryl-oxy, aryl-1-4C-alkoxy, trifluoromethyl, nitro, amino, mono- or di-1-4C-alkylamino, 1-4C-alkylcarbonylamino, 1-4Calkoxycarbonylamino, 1-4C-alkoxy-1-4C-alkoxycarbonylamino or sulfonyl,
R34 is hydrogen, 1-4C-alkyl, 1-4C-alkoxy, 1-4C-alkoxycarbonyl, halogen, trifluoromethyl or hydroxy,
R35 is hydrogen, 1-4C-alkyl, 1-4C-alkoxy, 1-4C-alkoxycarbonyl, halogen, trifluoromethyl or hydroxy,
R4 is hydrogen, halogen, 1-4C-alkyl, 3-7C-cycloalkyl, 3-7C-cycloalkyl-1-4C-alkyl, 1-4C-alkoxy-1-4C-alkyl, fluoro-1-4C-alkyl, fluoro-1-4C-alkoxy-1-4C-alkyl, hydroxy-1-4C-alkyl, hydroxy, 1-4C-alkoxy, 3-7C-cycloalkoxy, 3-7C-cycloalkyl-1-4C-alkoxy, 1-4C-alkoxy-1-4C-alkoxy, fluoro-1-4C-alkoxy, fluoro-1-4C-alkoxy-1-4C-alkoxy, hydroxy-1-4C-alkoxy, 2-4C-alkenyloxy or 2-4C-alkynyloxy
R5 is hydrogen, 1-4C-alkyl, 3-7C-cycloalkyl, 3-7C-cycloalkyl-1-4C-alkyl, 1-4C-alkoxy-1-4C-alkyl, fluoro-1-4C-alkyl, fluoro-1-4C-alkoxy- 1-4C-alkyl or hydroxy-1-4C-alkyl,
R6 is hydrogen, halogen, 1-4C-alkyl or fluoro-1-4C-alkyl,
R7 is hydrogen, halogen, 1-4C-alkyl or fluoro-1-4C-alkyl,
and wherein
aryl is phenyl or substituted phenyl with one, two or three identical or different substituents from the group of 1-4C-alkyl, 1-4C-alkoxy, carboxy, 1-4C-alkoxycarbonyl, halogen, trifluoromethyl, nitro, trifluoromethoxy, hydroxy and cyano,
and its salts.

10. A medicament comprising a compound as claimed in claim 1 and/or a pharmacologically acceptable salt thereof together with customary pharmaceutical auxiliaries and/or excipients.

11. The use of a compound as claimed in claim 1 and its pharmacologically acceptable salts for the manufacture of a medicament for the prevention and treatment of gastrointestinal disorders.

## Patentansprüche

1. Verbindung der Formel 1 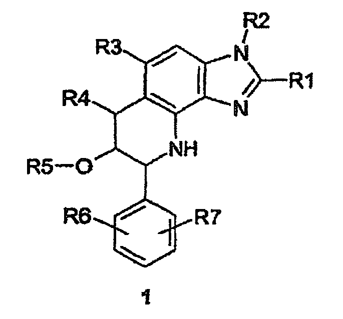 worin
R1 Wasserstoff, Halogen, 1-4C-Alkyl, 3-7C-Cycloalkyl, 3-7C-Cycloalkyl-1-4C-alkyl, 1-4C-Alkoxy, 1-4C-Alkoxy-1-4C-alkyl, 1-4C-Alkoxycarbonyl, 2-4C-Alkenyl, 2-4C-Alkinyl, Fluor-1-4C-alkyl, Fluor-1-4C-alkoxy-1-4C-alkyl oder Hydroxy-1-4C-alkyl bedeutet,
R2 Wasserstoff, 1-4C-Alkyl, 3-7C-Cycloalkyl, 3-7C-Cycloalkyl-1-4C-alkyl, 1-4C-Alkoxy-1-4C-alkyl, 2-4C-Alkenyl, 2-4C-Alkinyl, Fluor-1-4C-alkyl oder Hydroxy-1-4C-alkyl bedeutet,
R3 Halogen, Fluor-1-4C-alkyl, Carboxy, -CO-1-4C-Alkoxy, Hydroxy-1-4C-alkyl, 1-4C-Alkoxy-1-4C-alkyl, 1-4C-Alkoxy-1-4C-alkoxy-1-4C-alkyl, Fluor-1-4C-alkoxy-1-4C-alkyl, Cyano, die Gruppe -CO-NR31 R32, die Gruppe -SO₂-NR31 R32 oder die Gruppe Het bedeutet,
wobei
R31 Wasserstoff, Hydroxy, 1-7C-Alkyl, Hydroxy-1-4C-alkyl, 1-4C-Alkoxy-1-4C-alkyl, 3-7C-Cycloalkyl oder Amino bedeutet und
R32 Wasserstoff, 1-7C-Alkyl, Hydroxy-1-4C-alkyl oder 1-4C-Alkoxy-1-4C-alkyl bedeutet,
oder wobei
R31 und R32 gemeinsam unter Einschluß des Stickstoffatoms, an das beide gebunden sind, eine Pyrrolidino-, Piperidino-, Piperazino-, N-1-4C-Alkylpiperazino-, Morpholino-, Aziridino- oder Azetidinogruppe darstellen, und
Het einen durch R33, R34 und R35 substituierten heterocyclischen Rest aus der Gruppe bestehend aus Oxadiazol, Dihydrooxazol, Dihydroimidazol, Oxazol, Imidazol, Isoxazol, Dihydroisoxazol, Pyrazol und Tetrazol bedeutet,
wobei
R33 Wasserstoff, 1-4C-Alkyl, Hydroxy-1-4C-alkyl, 1-4C-Alkoxy, 2-4C-Alkenyloxy, 1-4C-Alkylcarbonyl, Carboxy, 1-4C-Alkoxycarbonyl, Carboxy-1-4C-alkyl, 1-4C-Alkoxycarbonyl-1-4C-alkyl, Halogen, Hydroxy, Aryl, Aryl-1-4C-alkyl, Aryl-oxy, Aryl-1-4C-alkoxy, Trifluormethyl, Nitro, Amino, Mono- oder Di-1-4C-alkylamino, 1-4C-Alkylcarbonylamino, 1-4C-Alkoxycarbonylamino, 1-4C-Alkoxy-1-4C-alkoxycarbonylamino oder Sulfonyl bedeutet,
R34 Wasserstoff, 1-4C-Alkyl, 1-4C-Alkoxy, 1-4C-Alkoxycarbonyl, Halogen, Trifluormethyl oder Hydroxy bedeutet,
R35 Wasserstoff, 1-4C-Alkyl, 1-4C-Alkoxy, 1-4C-Alkoxycarbonyl, Halogen, Trifluormethyl oder Hydroxy bedeutet,
R4 Wasserstoff, Halogen, 1-4C-Alkyl, 3-7C-Cycloalkyl, 3-7C-Cycloalkyl-1-4C-alkyl, 1-4C-Alkoxy-1-4C-alkyl, Fluor-1-4C-alkyl, Fluor-1-4C-alkoxy-1-4C-alkyl, Hydroxy-1-4C-alkyl, Hydroxy, 1-4C-Alkoxy, 3-7C-Cycloalkoxy, 3-7C-Cycloalkyl-1-4C-alkoxy, 1-4C-Alkoxy-1-4C-alkoxy, Fluor-1-4C-alkoxy, Fluor-1-4C-alkoxy-1-4C-alkoxy, Hydroxy-1-4C-alkoxy, 2-4C-Alkenyloxy oder 2-4C-Alkinyloxy bedeutet,
R5 Wasserstoff, 1-4C-Alkyl, 3-7C-Cycloalkyl, 3-7C-Cycloalkyl-1-4C-alkyl, 1-4C-Alkoxy-1-4C-alkyl, Fluor-1-4C-alkyl, Fluor-1-4C-alkoxy-1-4C-alkyl oder Hydroxy-1-4C-alkyl bedeutet,
R6 Wasserstoff, Halogen, 1-4C-Alkyl oder Fluor-1-4C-alkyl bedeutet,
R7 Wasserstoff, Halogen, 1-4C-Alkyl oder Fluor-1-4C-alkyl bedeutet,
und worin
Aryl für Phenyl oder substituiertes Phenyl mit einem, zwei oder drei gleichen oder verschiedenen Substituenten aus der Gruppe 1-4C-Alkyl, 1-4C-Alkoxy, Carboxy, 1-4C-Alkoxycarbonyl, Halogen, Trifluormethyl, Nitro, Trifluormethoxy, Hydroxy und Cyano steht,
und ihre Salze.

2. Verbindung der Formel 1 nach Anspruch 1, worin
R1 Wasserstoff, 1-4C-Alkyl oder 3-7C-Cycloalkyl bedeutet,
R2 Wasserstoff oder 1-4C-Alkyl bedeutet,
R3 Halogen, Fluor-1-4C-alkyl, Carboxy, -CO-1-4C-Alkoxy, Hydroxy-1-4C-alkyl, 1-4C-Alkoxy-1-4C-alkyl, 1-4C-Alkoxy-1-4C-alkoxy-1-4C-alkyl, Fluor-1-4C-alkoxy-1-4C-alkyl oder die Gruppe -CO-NR31 R32 bedeutet,
wobei
R31 Wasserstoff, Hydroxy, 1-7C-Alkyl, Hydroxy-1-4C-alkyl, 1-4C-Alkoxy-1-4C-alkyl oder 3-7C-Cycloalkyl bedeutet und
R32 Wasserstoff, 1-7C-Alkyl, Hydroxy-1-4C-alkyl oder 1-4C-Alkoxy-1-4C-alkyl bedeutet,
oder wobei
R31 und R32 gemeinsam unter Einschluß des Stickstoffatoms, an das beide gebunden sind, eine Pyrrolidino-, Piperidino-, Piperazino-, N-1-4C-Alkylpiperazino-, Morpholino-, Aziridino- oder Azetidinogruppe darstellen,
R4 Wasserstoff, Halogen, Hydroxy, 1-4C-Alkyl, 3-5C-Cycloalkyl, 1-4C-Alkoxy, 3-5C-Cycloalkoxy, 1-4C-Alkoxy-1-4C-alkoxy, Fluor-1-4C-alkoxy, Fluor-1-4C-alkoxy-1-4C-alkoxy, Hydroxy-1-4C-alkoxy, 2-4C-Alkenyloxy oder 2-4C-Alkinyloxy bedeutet,
R5 Wasserstoff, 1-4C-Alkyl oder Hydroxy-1-4C-alkyl bedeutet,
R6 Wasserstoff, Fluor, 1-4C-Alkyl oder Fluor-1-4C-alkyl bedeutet,
R7 Wasserstoff, Fluor, 1-4C-Alkyl oder Fluor-1-4C-alkyl bedeutet,
und ihre Salze.

3. Verbindung der Formel 1 nach Anspruch 1, worin
R1 Wasserstoff oder 1-4C-Alkyl bedeutet,
R2 1-4C-Alkyl bedeutet,
R3 Carboxy, -CO-1-4C-Alkoxy, Hydroxy-1-4C-alkyl, 1-4C-Alkoxy-1-4C-alkyl oder die Gruppe -CO-NR31 R32 bedeutet,
wobei
R31 Wasserstoff, 1-7C-Alkyl, Hydroxy-1-4C-alkyl oder 1-4C-Alkoxy-1-4C-alkyl bedeutet und
R32 Wasserstoff oder 1-7C-Alkyl bedeutet, oder wobei
R31 und R32 gemeinsam unter Einschluß des Stickstoffatoms, an das beide gebunden sind, eine Pyrrolidino-, Morpholino-, Aziridino- oder Azetidinogruppe darstellen,
R4 Wasserstoff, Halogen, Hydroxy, 1-4C-Alkyl, 3-5C-Cycloalkyl, 1-4C-Alkoxy, Fluor-1-4C-alkoxy, 1-4C-Alkoxy-1-4C-alkoxy, 2-4C-Alkenyloxy oder 2-4C-Alkinyloxy bedeutet,
R5 Wasserstoff bedeutet,
R6 Wasserstoff bedeutet,
R7 Wasserstoff bedeutet,
und ihre Salze.

4. Verbindung der Formel 1 nach Anspruch 1, worin
R1 Wasserstoff oder 1-4C-Alkyl bedeutet,
R2 1-4C-Alkyl bedeutet,
R3 Carboxy, -CO-1-4C-Alkoxy, Hydroxy-1-4C-alkyl, 1-4C-Alkoxy-1-4C-alkyl oder die Gruppe -CO-NR31 R32 bedeutet,
wobei
R31 Wasserstoff, 1-7C-Alkyl, Hydroxy-1-4C-alkyl oder 1-4C-Alkoxy-1-4C-alkyl bedeutet und
R32 Wasserstoff oder 1-7C-Alkyl bedeutet, oder wobei
R31 und R32 gemeinsam unter Einschluß des Stickstoffatoms, an das beide gebunden sind, eine Pyrrolidino-, Morpholino-, Aziridino- oder Azetidinogruppe darstellen,
R4 Wasserstoff, Halogen, Hydroxy, 1-4C-Alkyl, 3-5C-Cycloalkyl, 1-4C-Alkoxy, Fluor-1-4C-alkoxy, 1-4C-Alkoxy-1-4C-alkoxy, 2-4C-Alkenyloxy oder 2-4C-Alkinyloxy bedeutet,
R5 Wasserstoff oder 1-4C-Alkyl bedeutet,
R6 Wasserstoff bedeutet,
R7 Wasserstoff bedeutet,
und ihre Salze.

5. Verbindung der Formel 1 nach Anspruch 1, worin
R1 Wasserstoff oder 1-4C-Alkyl bedeutet,
R2 1-4C-Alkyl bedeutet,
R3 Carboxy, -CO-1-4C-Alkoxy oder die Gruppe -CO-NR31 R32 bedeutet,
wobei
R31 Wasserstoff, 1-4C-Alkyl, Hydroxy-1-4C-alkyl oder 1-4C-Alkoxy-1-4C-alkyl bedeutet und
R32 Wasserstoff oder 1-4C-Alkyl bedeutet, oder wobei
R31 und R32 gemeinsam unter Einschluß des Stickstoffatoms, an das beide gebunden sind, eine Pyrrolidino-, Morpholino-, Aziridino- oder Azetidinogruppe darstellen,
R4 Wasserstoff, Halogen, Hydroxy, 1-4C-Alkyl, 3-5C-Cycloalkyl, 1-4C-Alkoxy, Fluor-1-4C-alkoxy, 1-4C-Alkoxy-1-4C-alkoxy, 2-4C-Alkenyloxy oder 2-4C-Alkinyloxy bedeutet,
R5 Wasserstoff oder 1-4C-Alkyl bedeutet,
R6 Wasserstoff bedeutet,
R7 Wasserstoff bedeutet,
und ihre Salze.

6. Verbindung der Formel 1 nach Anspruch 1, worin
R1 1-4C-Alkyl bedeutet,
R2 1-4C-Alkyl bedeutet,
R3 -CO-NR31 R32 bedeutet,
wobei
R31 Wasserstoff oder 1-4C-Alkyl bedeutet und
R32 Wasserstoff oder 1-4C-Alkyl bedeutet,
R4 Wasserstoff, Hydroxy, 1-4C-Alkoxy, Fluor-1-4C-alkoxy, 1-4C-Alkoxy-1-4C-alkoxy oder 2-4C-Alkinyloxy bedeutet,
R5 Wasserstoff bedeutet,
R6 Wasserstoff bedeutet,
R7 Wasserstoff bedeutet,
und ihre Salze.

7. Verbindung der Formel 1 nach Anspruch 1, worin
R1 1-4C-Alkyl bedeutet,
R2 1-4C-Alkyl bedeutet,
R3 -CO-NR31 R32 bedeutet,
wobei
R31 Wasserstoff oder 1-4C-Alkyl bedeutet und
R32 Wasserstoff oder 1-4C-Alkyl bedeutet,
R4 Wasserstoff, Hydroxy, 1-4C-Alkoxy, Fluor-1-4C-alkoxy, 1-4C-Alkoxy-1-4C-alkoxy oder 2-4C-Alkinyloxy bedeutet,
R5 Wasserstoff oder 1-4C-Alkyl bedeutet,
R6 Wasserstoff bedeutet,
R7 Wasserstoff bedeutet,
und ihre Salze.

8. Verbindung der Formel 1 nach Anspruch 1, **gekennzeichnet durch** die Formel 1a 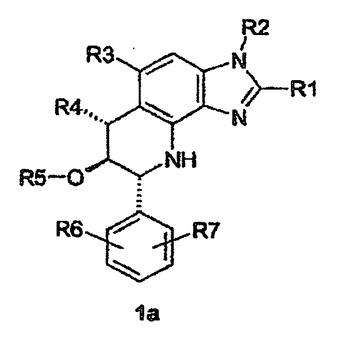 worin
R1 Wasserstoff, Halogen, 1-4C-Alkyl, 3-7C-Cycloalkyl, 3-7C-Cycloalkyl-1-4C-alkyl, 1-4C-Alkoxy, 1-4C-Alkoxy-1-4C-alkyl, 1-4C-Alkoxycarbonyl, 2-4C-Alkenyl, 2-4C-Alkinyl, Fluor-1-4C-alkyl, Fluor-1-4C-alkoxy-1-4C-alkyl oder Hydroxy-1-4C-alkyl bedeutet,
R2 Wasserstoff, 1-4C-Alkyl, 3-7C-Cycloalkyl, 3-7C-Cycloalkyl-1-4C-alkyl, 1-4C-Alkoxy-1-4C-alkyl, 2-4C-Alkenyl, 2-4C-Alkinyl, Fluor-1-4C-alkyl oder Hydroxy-1-4C-alkyl bedeutet,
R3 Halogen, Fluor-1-4C-alkyl, Carboxy, -CO-1-4C-Alkoxy, Hydroxy-1-4C-alkyl, 1-4C-Alkoxy-1-4C-alkyl, 1-4C-Alkoxy-1-4C-alkoxy-1-4C-alkyl, Fluor-1-4C-alkoxy-1-4C-alkyl, Cyano, die Gruppe -CO-NR31 R32, die Gruppe -SO₂-NR31R32 oder die Gruppe Het bedeutet,
wobei
R31 Wasserstoff, Hydroxy, 1-7C-Alkyl, Hydroxy-1-4C-alkyl, 1-4C-Alkoxy-1-4C-alkyl, 3-7C-Cycloalkyl oder Amino bedeutet und
R32 Wasserstoff, 1-7C-Alkyl, Hydroxy-1-4C-alkyl oder 1-4C-Alkoxy-1-4C-alkyl bedeutet,
oder wobei
R31 und R32 gemeinsam unter Einschluß des Stickstoffatoms, an das beide gebunden sind, eine Pyrrolidino-, Piperidino-, Piperazino-, N-1-4C-Alkylpiperazino-, Morpholino-, Aziridino- oder Azetidinogruppe darstellen, und Het einen **durch** R33, R34 und R35 substituierten heterocyclischen Rest aus der Gruppe bestehend aus Oxadiazol, Dihydrooxazol, Dihydroimidazol, Oxazol, Imidazol, Isoxazol, Dihydroisoxazol, Pyrazol und Tetrazol bedeutet,
wobei
R33 Wasserstoff, 1-4C-Alkyl, Hydroxy-1-4C-alkyl, 1-4C-Alkoxy, 2-4C-Alkenyloxy, 1-4C-Alkylcarbonyl, Carboxy, 1-4C-Alkoxycarbonyl, Carboxy-1-4C-alkyl, 1-4C-Alkoxycarbonyl-1-4C-alkyl, Halogen, Hydroxy, Aryl, Aryl-1-4C-alkyl, Aryl-oxy, Aryl-1-4C-alkoxy, Trifluormethyl, Nitro, Amino, Mono- oder Di-1-4C-alkylamino, 1-4C-Alkylcarbonylamino, 1-4C-Alkoxycarbonylamino, 1-4C-Alkoxy-1-4C-alkoxycarbonylamino oder Sulfonyl bedeutet,
R34 Wasserstoff, 1-4C-Alkyl, 1-4C-Alkoxy, 1-4C-Alkoxycarbonyl, Halogen, Trifluormethyl oder Hydroxy bedeutet,
R35 Wasserstoff, 1-4C-Alkyl, 1-4C-Alkoxy, 1-4C-Alkoxycarbonyl, Halogen, Trifluormethyl oder Hydroxy bedeutet,
R4 Wasserstoff, Halogen, 1-4C-Alkyl, 3-7C-Cycloalkyl, 3-7C-Cycloalkyl-1-4C-alkyl, 1-4C-Alkoxy-1-4C-alkyl, Fluor-1-4C-alkyl, Fluor-1-4C-alkoxy-1-4C-alkyl, Hydroxy-1-4C-alkyl, Hydroxy, 1-4C-Alkoxy, 3-7C-Cycloalkoxy, 3-7C-Cycloalkyl-1-4C-alkoxy, 1-4C-Alkoxy-1-4C-alkoxy, Fluor-1-4C-alkoxy, Fluor-1-4C-alkoxy-1-4C-alkoxy, Hydroxy-1-4C-alkoxy, 2-4C-Alkenyloxy oder 2-4C-Alkinyloxy bedeutet,
R5 Wasserstoff, 1-4C-Alkyl, 3-7C-Cycloalkyl, 3-7C-Cycloalkyl-1-4C-alkyl, 1-4C-Alkoxy-1-4C-alkyl, Fluor-1-4C-alkyl, Fluor-1-4C-alkoxy-1-4C-alkyl oder Hydroxy-1-4C-alkyl bedeutet,
R6 Wasserstoff, Halogen, 1-4C-Alkyl oder Fluor-1-4C-alkyl bedeutet,
R7 Wasserstoff, Halogen, 1-4C-Alkyl oder Fluor-1-4C-alkyl bedeutet, und worin
Aryl für Phenyl oder substituiertes Phenyl mit einem, zwei oder drei gleichen oder verschiedenen Substituenten aus der Gruppe 1-4C-Alkyl, 1-4C-Alkoxy, Carboxy, 1-4C-Alkoxycarbonyl, Halogen, Trifluormethyl, Nitro, Trifluormethoxy, Hydroxy und Cyano steht,
und ihre Salze.

9. Verbindung der Formel 1b 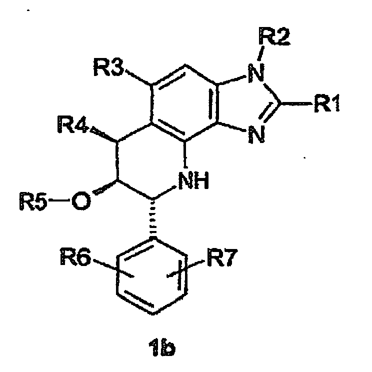 worin
R1 Wasserstoff, Halogen, 1-4C-Alkyl, 3-7C-Cycloalkyl, 3-7C-Cycloalkyl-1-4C-alkyl, 1-4C-Alkoxy, 1-4C-Alkoxy-1-4C-alkyl, 1-4C-Alkoxycarbonyl, 2-4C-Alkenyl, 2-4C-Alkinyl, Fluor-1-4C-alkyl, Fluor-1-4C-alkoxy-1-4C-alkyl oder Hydroxy-1-4C-alkyl bedeutet,
R2 Wasserstoff, 1-4C-Alkyl, 3-7C-Cycloalkyl, 3-7C-Cycloalkyl-1-4C-alkyl, 1-4C-Alkoxy-1-4C-alkyl, 2-4C-Alkenyl, 2-4C-Alkinyl, Fluor-1-4C-alkyl oder Hydroxy-1-4C-alkyl bedeutet,
R3 Halogen, Fluor-1-4C-alkyl, Carboxy, -CO-1-4C-Alkoxy, Hydroxy-1-4C-alkyl, 1-4C-Alkoxy-1-4C-alkyl, 1-4C-Alkoxy-1-4C-alkoxy-1-4C-alkyl, Fluor-1-4C-alkoxy-1-4C-alkyl, Cyano, die Gruppe -CO-NR31 R32, die Gruppe -SO₂-NR31 R32 oder die Gruppe Het bedeutet,
wobei
R31 Wasserstoff, Hydroxy, 1-7C-Alkyl, Hydroxy-1-4C-alkyl, 1-4C-Alkoxy-1-4C-alkyl, 3-7C-Cycloalkyl oder Amino bedeutet und
R32 Wasserstoff, 1-7C-Alkyl, Hydroxy-1-4C-alkyl oder 1-4C-Alkoxy-1-4C-alkyl bedeutet,
oder wobei
R31 und R32 gemeinsam unter Einschluß des Stickstoffatoms, an das beide gebunden sind, eine Pyrrolidino-, Piperidino-, Piperazino-, N-1-4C-Alkylpiperazino-, Morpholino-, Aziridino- oder Azetidinogruppe darstellen, und
Het einen durch R33, R34 und R35 substituierten heterocyclischen Rest aus der Gruppe bestehend aus Oxadiazol, Dihydrooxazol, Dihydroimidazol, Oxazol, Imidazol, Isoxazol, Dihydroisoxazol, Pyrazol und Tetrazol bedeutet,
wobei
R33 Wasserstoff, 1-4C-Alkyl, Hydroxy-1-4C-alkyl, 1-4C-Alkoxy, 2-4C-Alkenyloxy, 1-4C-Alkylcarbonyl, Carboxy, 1-4C-Alkoxycarbonyl, Carboxy-1-4C-alkyl, 1-4C-Alkoxycarbonyl-1-4C-alkyl, Halogen, Hydroxy, Aryl, Aryl-1-4C-alkyl, Aryl-oxy, Aryl-1-4C-alkoxy, Trifluormethyl, Nitro, Amino, Mono- oder Di-1-4C-alkylamino, 1-4C-Alkylcarbonylamino, 1-4C-Alkoxycarbonylamino, 1-4C-Alkoxy-1-4C-alkoxycarbonylamino oder Sulfonyl bedeutet,
R34 Wasserstoff, 1-4C-Alkyl, 1-4C-Alkoxy, 1-4C-Alkoxycarbonyl, Halogen, Trifluormethyl oder Hydroxy bedeutet,
R35 Wasserstoff, 1-4C-Alkyl, 1-4C-Alkoxy, 1-4C-Alkoxycarbonyl, Halogen, Trifluormethyl oder Hydroxy bedeutet,
R4 Wasserstoff, Halogen, 1-4C-Alkyl, 3-7C-Cycloalkyl, 3-7C-Cycloalkyl-1-4C-alkyl, 1-4C-Alkoxy-1-4C-alkyl, Fluor-1-4C-alkyl, Fluor-1-4C-alkoxy-1-4C-alkyl, Hydroxy-1-4C-alkyl, Hydroxy, 1-4C-Alkoxy, 3-7C-Cycloalkoxy, 3-7C-Cycloalkyl-1-4C-alkoxy, 1-4C-Alkoxy-1-4C-alkoxy, Fluor-1-4C-alkoxy, Fluor-1-4C-alkoxy-1-4C-alkoxy, Hydroxy-1-4C-alkoxy, 2-4C-Alkenyloxy oder 2-4C-Alkinyloxy bedeutet,
R5 Wasserstoff, 1-4C-Alkyl, 3-7C-Cycloalkyl, 3-7C-Cycloalkyl-1-4C-alkyl, 1-4C-Alkoxy-1-4C-alkyl, Fluor-1-4C-alkyl, Fluor-1-4C-alkoxy-1-4C-alkyl oder Hydroxy-1-4C-alkyl bedeutet,
R6 Wasserstoff, Halogen, 1-4C-Alkyl oder Fluor-1-4C-alkyl bedeutet,
R7 Wasserstoff, Halogen, 1-4C-Alkyl oder Fluor-1-4C-alkyl bedeutet,
und worin
Aryl für Phenyl oder substituiertes Phenyl mit einem, zwei oder drei gleichen oder verschiedenen Substituenten aus der Gruppe 1-4C-Alkyl, 1-4C-Alkoxy, Carboxy, 1-4C-Alkoxycarbonyl, Halogen, Trifluormethyl, Nitro, Trifluormethoxy, Hydroxy und Cyano steht,
und ihre Salze.

10. Arzneimittel enthaltend eine Verbindung nach Anspruch 1 und/oder ein pharmakologisch verträgliches Salz davon zusammen mit üblichen pharmazeutischen Hilfs- und/oder Trägerstoffen.

11. Verwendung einer Verbindung nach Anspruch 1 und ihrer pharmakologisch verträglichen Salze zur Herstellung eines Arzneimittels zur Verhütung und Behandlung gastrointestinaler Krankheiten.

## Revendications

1. Composé de formule 1, 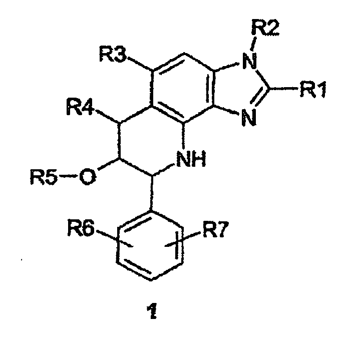 dans laquelle :
R1 est hydrogène, halogène, C1-4-alkyle, C3-7-cycloalkyle, C3-7-cycloalkyl-C1-4-alkyle, C1-4-alcoxy, C1-4-alcoxy-C1-4-alkyle, C1-4-alcoxycarbonyle, C2-4-alcényle, C2-4-alcynyle, fluoro-C1-4-alkyle, fluoro-C1-4-alcoxy-C1-4-alkyle ou hydroxy-C1-4-alkyle,
R2 est hydrogène, C1-4-alkyle, C3-7-cycloalkyle, C3-7-cycloalkyl-C1-4-alkyle, C1-4-alcoxy-C1-4-alkyle, C2-4-alcényle, C2-4-alcynyle, fluoro-C1-4-alkyle ou hydroxy-C1-4-alkyle,
R3 est halogène, fluoro-C1-4-alkyle, carboxyle, -CO-C1-4-alcoxy, hydroxy-C1-4-alkyle, C1-4-alcoxy-C1-4-alkyle, C1-4-alcoxy-C1-4-alcoxy-C1-4-alkyle, fluoro-C1-4-alcoxy-C1-4-alkyle, cyano, le groupement -CO-NR31 R32, le groupement SO₂-NR31R32 ou le groupement Het,
où
R31 est hydrogène, hydroxy, C1-7-alkyle, hydroxy-C1-4-alkyle, C1-4-alcoxy-C1-4-alkyle, C3-7-cycloalkyle ou amino, et
R32 est hydrogène, C1-7-alkyle, hydroxy-C1-4-alkyle ou C1-4-alcoxy-C1-4-alkyle,
ou, où
R31 et R32 sont, conjointement, y compris avec l'atome d'azote auquel les deux sont liés, un groupement pyrrolidino, pipéridino, pipérazino, N-C1-4-alkylpipérazino, morpholino, aziridino ou azétidino, et
Het est un résidu hétérocyclique, substitué par R33, R34 et R35, choisi dans le groupe constitué d'oxadiazole, de dihydrooxazole, de dihydroimidazole, d'oxazole, d'imidazole, d'isoxazole, de dihydroisoxazole, de pyrazole et de tétrazole,
où
R33 est hydrogène, C1-4-alkyle, hydroxy-C1-4-alkyle, C1-4-alcoxy, C2-4-alcényloxy, C1-4-alkylcarbonyle, carboxy, C1-4-alcoxycarbonyle, carboxy-C1-4-alkyle, C1-4-alcoxycarbonyl-C1-4-alkyle, halogène, hydroxy, aryle, aryl-C1-4-alkyle, aryloxy, aryl-C1-4-alcoxy, trifluorométhyle, nitro, amino, mono- ou di-C1-4-alkylamino, C1-4-alkylcarbonylamino, C1-4-alcoxycarbonylamino, C1-4-alcoxy-C1-4-alcoxycarbonylamino ou sulfonyle,
R34 est hydrogène, C1-4-alkyle, C1-4-alcoxy, C1-4-alcoxycarbonyle, halogène, trifluorométhyle ou hydroxy,
R35 est hydrogène, C1-4-alkyle, C1-4-alcoxy, C1-4-alcoxycarbonyle, halogène, trifluorométhyle ou hydroxy,
R4 est hydrogène, halogène, C1-4-alkyle, C3-7-cycloalkyle, C3-7-cycloalkyl-C1-4-alkyle, C1-4-alcoxy-C1-4-alkyle, fluoro-C1-4-alkyle, fluoro-C1-4-alcoxy-C1-4-alkyle, hydroxy-C1-4-alkyle, hydroxy, C1-4-alcoxy, C3-7-cycloalcoxy, C3-7-cycloalkyl-C1-4-alcoxy, C1-4-alcoxy-C1-4-alcoxy, fluoro-C1-4-alcoxy, fluoro-C1-4-alcoxy-C1-4-alcoxy, hydroxy-C1-4-alcoxy, C2-4-alcényloxy ou C2-4-alcynyloxy,
R5 est hydrogène, C1-4-alkyle, C3-7-cycloalkyle, C3-7-cycloalkyl-C1-4-alkyle, C1-4-alcoxy-C1-4-alkyle, fluoro-C1-4-alkyle, fluoro-C1-4-alcoxy-C1-4-alkyle ou hydroxy-C1-4-alkyle,
R6 est hydrogène, halogène, C1-4-alkyle ou fluoro-C1-4-alkyle,
R7 est hydrogène, halogène, C1-4-alkyle ou fluoro-C1-4-alkyle,
et où
aryle est phényle ou phényle substitué par un, deux ou trois substituants identiques ou différents choisis dans le groupe comprenant C1-4-alkyle, C1-4-alcoxy, carboxy, C1-4-alcoxycarbonyle, halogène, trifluorométhyle, nitro, trifluorométhoxy, hydroxy et cyano,
et ses sels.

2. Composé de formule 1 selon la revendication 1, **caractérisé en ce que** :
R1 est hydrogène, C1-4-alkyle ou C3-7-cycloalkyle,
R2 est hydrogène ou C1-4-alkyle,
R3 est halogène, fluoro-C1-4-alkyle, carboxyle, -CO-C1-4-alcoxy, hydroxy-C1-4-alkyle, C1-4-alcoxy-C1-4-alkyle, C1-4-alcoxy-C1-4-alcoxy-C1-4-alkyle, fluoro-C1-4-alcoxy-C1-4-alkyle ou le groupement -CO-NR31 R32,
où
R31 est hydrogène, hydroxy, C1-7-alkyle, hydroxy-C1-4-alkyle, C1-4-alcoxy-C1-4-alkyle ou C3-7-cycloalkyle,
R32 est hydrogène, C1-7-alkyle, hydroxy-C1-4-alkyle ou C1-4-alcoxy-C1-4-alkyle,
ou, où .
R31 et R32 sont, conjointement, y compris avec l'atome d'azote auquel les deux sont liés, un groupement pyrrolidino, pipéridino, pipérazino, N-C1-4-alkylpipérazino, morpholino, aziridino ou azétidino,
R4 est hydrogène, halogène, hydroxy, C1-4-alkyle, C3-5-cycloalkyle, C1-4-alcoxy, C3-5-cycloalcoxy, C1-4-alcoxy-C1-4-alcoxy, fluoro-C1-4-alcoxy, fluoro-C1-4-alcoxy-C1-4-alcoxy, hydroxy-C1-4-alcoxy, C2-4-alcényloxy ou C2-4-alcynyloxy,
R5 est hydrogène, C1-4-alkyle ou hydroxy-C1-4-alkyle,
R6 est hydrogène, fluoro, C1-4-alkyle ou fluoro-C1-4-alkyle,
R7 est hydrogène, fluoro, C1-4-alkyle ou fluoro-C1-4-alkyle,
et ses sels.

3. Composé de formule 1 selon la revendication 1, **caractérisé en ce que** :
R1 est hydrogène ou C1-4-alkyle,
R2 est C1-4-alkyle,
R3 est carboxyle, -CO-C1-4-alcoxy, hydroxy-C1-4-alkyle, C1-4-alcoxy-C1-4-alkyle ou le groupement -CO-NR31 R32,
où
R31 est hydrogène, C1-7-alkyle, hydroxy-C1-4-alkyle ou C1-4-alcoxy-C1-4-alkyle et
R32 est hydrogène ou C1-7-alkyle,
ou, où
R31 et R32 sont, conjointement, y compris avec l'atome d'azote auquel les deux sont liés, un groupement pyrrolidino, morpholino, aziridino ou azétidino,
R4 est hydrogène, halogène, hydroxy, C1-4-alkyle, C3-5-cycloalkyle, C1-4-alcoxy, fluoro-C1-4-alcoxy, C1-4-alcoxy-C1-4-alcoxy, C2-4-alcényloxy ou C2-4-alcynyloxy,
R5 est hydrogène,
R6 est hydrogène,
R7 est hydrogène,
et ses sels.

4. Composé de formule 1 selon la revendication 1, **caractérisé en ce que** :
R1 est hydrogène ou C1-4-alkyle,
R2 est C1-4-alkyle,
R3 est carboxyle, -CO-C1-4-alcoxy, hydroxy-C1-4-alkyle, C1-4-alcoxy-C1-4-alkyle ou le groupement -CO-NR31 R32,
où
R31 est hydrogène, C1-7-alkyle, hydroxy-C1-4-alkyle ou C1-4-alcoxy-C1-4-alkyle et
R32 est hydrogène ou C1-7-alkyle,
ou, où
R31 et R32 sont, conjointement, y compris avec l'atome d'azote auquel les deux sont liés, un groupement pyrrolidino, morpholino, aziridino ou azétidino,
R4 est hydrogène, halogène, hydroxy, C1-4-alkyle, C3-5-cycloalkyle, C1-4-alcoxy, fluoro-C1-4-alcoxy, C1-4-alcoxy-C1-4-alcoxy, C2-4-alcényloxy ou C2-4-alcynyloxy,
R5 est hydrogène ou C1-4-alkyle,
R6 est hydrogène,
R7 est hydrogène,
et ses sels.

5. Composé de formule 1 selon la revendication 1, **caractérisé en ce que** :
R1 est hydrogène ou C1-4-alkyle,
R2 est C1-4-alkyle,
R3 est carboxyle, -CO-C1-4-alcoxy ou le groupement -CO-NR31 R32,
où
R31 est hydrogène, C1-4-alkyle, hydroxy-C1-4-alkyle ou C1-4-alcoxy-C1-4-alkyle et
R32 est hydrogène ou C1-4-alkyle,
ou, où
R31 et R32 sont, conjointement, y compris avec l'atome d'azote auquel les deux sont liés, sont un groupement pyrrolidino, morpholino, aziridino ou azétidino,
R4 est hydrogène, halogène, hydroxy, C1-4-alkyle, C3-5-cycloalkyle, C1-4-alcoxy, fluoro-C1-4-alcoxy, C1-4-alcoxy-C1-4-alcoxy, C2-4-alcényloxy ou C2-4-alcynyloxy,
R5 est hydrogène ou C1-4-alkyle,
R6 est hydrogène,
R7 est hydrogène,
et ses sels.

6. Composé de formule 1 selon la revendication 1, **caractérisé en ce que** :
R1 est C1-4-alkyle,
R2 est C1-4-alkyle,
R3 est -CO-NR31R32,
où
R31 est hydrogène ou C1-4-alkyle,
R32 est hydrogène ou C1-4-alkyle,
R4 est hydrogène, hydroxy, C1-4-alcoxy, fluoro-C1-4-alcoxy, C1-4-alcoxy-C1-4-alcoxy ou C2-4-alcynyloxy,
R5 est hydrogène,
R6 est hydrogène,
R7 est hydrogène,
et ses sels.

7. Composé de formule 1 selon la revendication 1, **caractérisé en ce que** :
R1 est C1-4-alkyle,
R2 est C1-4-alkyle,
R3 est -CO-NR31 R32,
où
R31 est hydrogène ou C1-4-alkyle,
R32 est hydrogène ou C1-4-alkyle,
R4 est hydrogène, hydroxy, C1-4-alcoxy, fluoro-C1-4-alcoxy, C1-4-alcoxy-C1-4-alcoxy ou C2-4-alcynyloxy,
R5 est hydrogène ou C1-4-alkyle,
R6 est hydrogène,
R7 est hydrogène,
et ses sels.

8. Composé de formule 1 selon la revendication 1, **caractérisé par** la formule 1a, 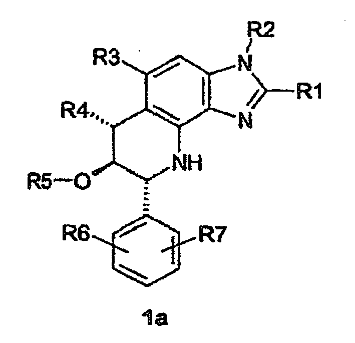 dans laquelle :
R1 est hydrogène, halogène, C1-4-alkyle, C3-7-cycloalkyle, C3-7-cycloalkyl-C1-4-alkyle, C1-4-alcoxy, C1-4-alcoxy-C1-4-alkyle, C1-4-alcoxycarbonyle, C2-4-alcényle, C2-4-alcynyle, fluoro-C1-4-alkyle, fluoro-C1-4-alcoxy-C1-4-alkyle ou hydroxy-C1-4-alkyle,
R2 est hydrogène, C1-4-alkyle, C3-7-cycloalkyle, C3-7-cycloalkyl-C1-4-alkyle, C1-4-alcoxy-C1-4-alkyle, C2-4-alcényle, C2-4-alcynyle, fluoro-C1-4-alkyle ou hydroxy-C1-4-alkyle,
R3 est halogène, fluoro-C1-4-alkyle, carboxyle, -CO-C1-4-alcoxy, hydroxy-C1-4-alkyle, C1-4-alcoxy-C1-4-alkyle, C1-4-alcoxy-C1-4-alcoxy-C1-4-alkyle, fluoro-C1-4-alcoxy-C1-4-alkyle, cyano, le groupement -CO-NR31 R32, le groupement SO₂-NR31 R32 ou le groupement Het,
où
R31 est hydrogène, hydroxy, C1-7-alkyle, hydroxy-C1-4-alkyle, C1-4-alcoxy-C1-4-alkyle, C3-7-cycloalkyle ou amino, et
R32 est hydrogène, C1-7-alkyle, hydroxy-C1-4-alkyle ou C1-4-alcoxy-C1-4-alkyle,
ou, où
R31 et R32 sont, conjointement, y compris avec l'atome d'azote auquel les deux sont liés, un groupement pyrrolidino, pipéridino, pipérazino, N-C1-4-alkylpipérazino, morpholino, aziridino ou azétidino, et
Het est un résidu hétérocyclique, substitué par R33, R34 et R35, choisi dans le groupe constitué d'oxadiazole, de dihydrooxazole, de dihydroimidazole, d'oxazole, d'imidazole, d'isoxazole, de dihydroisoxazole, de pyrazole et de tétrazole,
où
R33 est hydrogène, C1-4-alkyle, hydroxy-C1-4-alkyle, C1-4-alcoxy, C2-4-alcényloxy, C1-4-alkylcarbonyle, carboxy, C1-4-alcoxycarbonyle, carboxy-C1-4-alkyle, C1-4-alcoxycarbonyl-C1-4-alkyle, halogène, hydroxy, aryle, aryl-C1-4-alkyle, aryloxy, aryl-C1-4-alcoxy, trifluorométhyle, nitro, amino, mono- ou di-C1-4-alkylamino, C1-4-alkylcarbonylamino, C1-4-alcoxycarbonylamino, C1-4-alcoxy-C1-4-alcoxycarbonylamino ou sulfonyle,
R34 est hydrogène, C1-4-alkyle, C1-4-alcoxy, C1-4-alcoxycarbonyle, halogène, trifluorométhyle ou hydroxy,
R35 est hydrogène, C1-4-alkyle, C1-4-alcoxy, Cl-4-alcoxycarbonyle, halogène, trifluorométhyle ou hydroxy,
R4 est hydrogène, halogène, C1-4-alkyle, C3-7-cycloalkyle, C3-7-cycloalkyl-C1-4-alkyle, C1-4-alcoxy-C1-4-alkyle, fluoro-C1-4-alkyle, fluoro-C1-4-alcoxy-C1-4-alkyle, hydroxy-C1-4-alkyle, hydroxy, C1-4-alcoxy, C3-7-cycloalcoxy, C3-7-cycloalkyl-C1-4-alcoxy, C1-4-alcoxy-C1-4-alcoxy, fluoro-C1-4-alcoxy, fluoro-C1-4-alcoxy-C1-4-alcoxy, hydroxy-C1-4-alcoxy, C2-4-alcényloxy ou C2-4-alcynyloxy,
R5 est hydrogène, C1-4-alkyle, C3-7-cycloalkyle, C3-7-cycloalkyl-C1-4-alkyle, C1-4-alcoxy-C1-4-alkyle, fluoro-C1-4-alkyle, fluoro-C1-4-alcoxy-C1-4-alkyle ou hydroxy-C1-4-alkyle,
R6 est hydrogène, halogène, C1-4-alkyle ou fluoro-C1-4-alkyle,
R7 est hydrogène, halogène, C1-4-alkyle ou fluoro-C1-4-alkyle,
et où
aryle est phényle ou phényle substitué par un, deux ou trois substituants identiques ou différents choisis dans le groupe comprenant C1-4-alkyle, C1-4-alcoxy, carboxy, C1-4-alcoxycarbonyle, halogène, trifluorométhyle, nitro, trifluorométhoxy, hydroxy et cyano,
et ses sels.

9. Composé de formule 1 b, 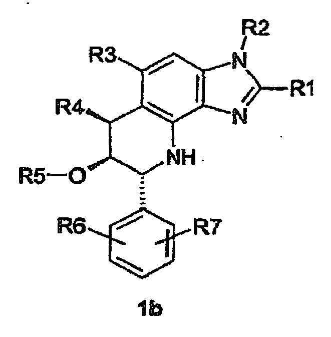 dans laquelle :
R1 est hydrogène, halogène, C1-4-alkyle, C3-7-cycloalkyle, C3-7-cycloalkyl-C1-4-alkyle, C1-4-alcoxy, C1-4-alcoxy-C1-4-alkyle, C1-4-alcoxycarbonyle, C2-4-alcényle, C2-4-alcynyle, fluoro-C1-4-alkyle, fluoro-C1-4-alcoxy-C1-4-alkyle ou hydroxy-C1-4-alkyle,
R2 est hydrogène, C1-4-alkyle, C3-7-cycloalkyle, C3-7-cycloalkyl-C1-4-alkyle, C1-4-alcoxy-C1-4-alkyle, C2-4-alcényle, C2-4-alcynyle, fluoro-C1-4-alkyle ou hydroxy-C1-4-alkyle,
R3 est halogène, fluoro-C1-4-alkyle, carboxyle, -CO-C1-4-alcoxy, hydroxy-C1-4-alkyle, C1-4-alcoxy-C1-4-alkyle, C1-4-alcoxy-C1-4-alcoxy-C1-4-alkyle, fluoro-C1-4-alcoxy-C1-4-alkyle, cyano, le groupement -CO-NR31 R32, le groupement SO₂-NR31 R32 ou le groupement Het,
où
R31 est hydrogène, hydroxy, C1-7-alkyle, hydroxy-C1-4-alkyle, C1-4-alcoxy-C1-4-alkyle, C3-7-cycloalkyle ou amino, et
R32 est hydrogène, C1-7-alkyle, hydroxy-C1-4-alkyle ou C1-4-alcoxy-C1-4-alkyle,
ou, où
R31 et R32 sont, conjointement, y compris avec l'atome d'azote auquel les deux sont liés, un groupement pyrrolidino, pipéridino, pipérazino, N-C1-4-alkylpipérazino, morpholino, aziridino ou azétidino, et
Het est un résidu hétérocyclique, substitué par R33, R34 et R35, choisi dans le groupe constitué d'oxadiazole, de dihydrooxazole, de dihydroimidazole, d'oxazole, d'imidazole, d'isoxazole, de dihydroisoxazole, de pyrazole et de tétrazole,
où
R33 est hydrogène, C1-4-alkyle, hydroxy-C1-4-alkyle, C1-4-alcoxy, C2-4-alcényloxy, C1-4-alkylcarbonyle, carboxy, C1-4-alcoxycarbonyle, carboxy-C1-4-alkyle, C1-4-alcoxycarbonyl-C1-4-alkyle, halogène, hydroxy, aryle, aryl-C1-4-alkyle, aryloxy, aryl-C1-4-alcoxy, trifluorométhyle, nitro, amino, mono- ou di-C1-4-alkylamino, C1-4-alkylcarbonylamino, C1-4-alcoxycarbonylamino, C1-4-alcoxy-C1-4-alcoxycarbonylamino ou sulfonyle,
R34 est hydrogène, C1-4-alkyle, C1-4-alcoxy, C1-4-alcoxycarbonyle, halogène, trifluorométhyle ou hydroxy,
R35 est hydrogène, C1-4-alkyle, C1-4-alcoxy, C1-4-alcoxycarbonyle, halogène, trifluorométhyle ou hydroxy,
R4 est hydrogène, halogène, C1-4-alkyle, C3-7-cycloalkyle, C3-7-cycloalkyl-C1-4-alkyle, C1-4-alcoxy-C1-4-alkyle, fluoro-C1-4-alkyle, fluoro-C1-4-alcoxy-C1-4-alkyle, hydroxy-C1-4-alkyle, hydroxy, C1-4-alcoxy, C3-7-cycloalcoxy, C3-7-cycloalkyl-C1-4-alcoxy, C1-4-alcoxy-C1-4-alcoxy, fluoro-C1-4-alcoxy, fluoro-C1-4-alcoxy-C1-4-alcoxy, hydroxy-C1-4-alcoxy, C2-4-alcényloxy ou C2-4-alcynyloxy,
R5 est hydrogène, C1-4-alkyle, C3-7-cycloalkyle, C3-7-cycloalkyl-C1-4-alkyle, C1-4-alcoxy-C1-4-alkyle, fluoro-C1-4-alkyle, fluoro-C1-4-alcoxy-C1-4-alkyle ou hydroxy-C1-4-alkyle,
R6 est hydrogène, halogène, C1-4-alkyle ou fluoro-C1-4-alkyle,
R7 est hydrogène, halogène, C1-4-alkyle ou fluoro-C1-4-alkyle,
et où
aryle est phényle ou phényle substitué par un, deux ou trois substituants identiques ou différents choisis dans le groupe comprenant C1-4-alkyle, C1-4-alcoxy, carboxy, C1-4-alcoxycarbonyle, halogène, trifluorométhyle, nitro, trifluorométhoxy, hydroxy et cyano,
et ses sels.

10. Médicament comprenant un composé selon la revendication 1 et/ou un sel pharmacologiquement acceptable de celui-ci conjointement avec les auxiliaires et/ou les excipients pharmaceutiques habituels.

11. Utilisation d'un composé selon la revendication 1 et de ses sels pharmacologiquement acceptables pour la fabrication d'un médicament destiné à la prévention et au traitement de troubles gastro-intestinaux.
